# EUROPEAN PATENT SPECIFICATION

(11) **EP 3 556 775 B1**
(45) Date of publication and mention of the grant of the patent: **17.11.2021**
(21) Application number: 19161885.9
(22) Date of filing: 26.01.2015
(51) Int. Cl.: C07K 16/28, A61K 39/395, A61P 35/00, A61P 35/02

(54) **ANTI-LAG-3 ANTIBODIES TO TREAT HEMATOLOGICAL MALIGNANCIES**
ANTI-LAG-3-ANTIKÖRPER ZUR BEHANDLUNG VON HÄMATOLOGISCHEN MALIGNOMEN
ANTICORPS ANTI-LAG-3 POUR TRAITER DES MALIGNITÉS HÉMATOLOGIQUES

(30) Priority: 28.01.2014 US 201461932589 P
(43) Date of publication of application: 23.10.2019
(62) Divisional of application: 15705137.6
(73) Proprietor: Bristol-Myers Squibb Company, Princeton, NJ 08543 (US)
(72) Inventor: GUTIERREZ, Andres A., Princeton, NJ 08543 (US); GROSSO, Joseph, Princeton, NJ 08543 (US); HILL, Christopher Mark, Seattle, WA 98102 (US); SELBY, Mark, Redwood City, CA 94063 (US); LEWIS, Katherine E., Seattle, WA 98102 (US)
(74) Representative: Mewburn Ellis LLP

(56) References cited:
- WO-A1-2014/008218
- WO-A1-2015/042246
- WO-A2-2010/019570
- Anonymous: "A Phase 1 Dose Escalation and Cohort Expansion Study of the Safety, Tolerability, and Efficacy of Anti-LAG-3 Monoclonal Antibody (BMS-986016) Administered Alone and in Combination With Anti-PD-1 Monoclonal Antibody (Nivolumab, BMS-936558) in Advanced Solid Tumors", ClinicalTrials.gov archive, 23 January 2014 (2014-01-23), pages 1-7, XP055195481, Retrieved from the Internet: URL:https://clinicaltrials.gov/archive/NCT 01968109/2014_01_23 [retrieved on 2015-06-12]
- Anonymous: "A Phase 1 Dose Escalation and Cohort Expansion Study of the Safety, Tolerability, and Efficacy of Anti-LAG-3 (BMS-986016) in Relapsed or Refractory Chronic Lymphocytic Leukemia and Lymphomas and Multiple Myeloma", ClinicalTrials.gov Archive, 20 November 2014 (2014-11-20), pages 1-7, XP055195982, Retrieved from the Internet: URL:https://clinicaltrials.gov/archive/NCT 02061761/2014_11_20 [retrieved on 2015-06-16]
- LIPSON: "Initial experience administering BMS-986016, a monoclonalantibody that targets lymphocyte activation gene (LAG)-3, aloneand in combination with nivolumab to patients with hematologicand solid malignancies", JOURNAL FOR IMMUNOTHERAPY OF CANCER, vol. 4, no. 1, P232, 16 November 2016 (2016-11-16), pages 129-130, XP021241441,

## Description

### BACKGROUND

Lymphocyte activation gene-3 (LAG-3; CD223) is a type I transmembrane protein that is expressed on the cell surface of activated CD4⁺ and CD8⁺ T cells and subsets of NK and dendritic cells (Triebel F, et al., J. Exp. Med. 1990; 171:1393-1405; Workman CJ, et al., J. Immunol. 2009; 182(4):1885-91). LAG-3 is closely related to CD4, which is a co-receptor for T helper cell activation. Both molecules have four extracellular Ig-like domains and require binding to their ligand, major histocompatibility complex (MHC) class II, for their functional activity. In contrast to CD4, LAG-3 is only expressed on the cell surface of activated T cells and its cleavage from the cell surface terminates LAG-3 signaling. LAG-3 can also be found as a soluble protein but it does not bind to MHC class II and its function is unknown.

It has been reported that LAG-3 plays an important role in promoting regulatory T cell (Treg) activity and in negatively regulating T cell activation and proliferation (Workman CJ, et al., J. Immunol. 2005; 174:688-695). Both natural and induced Treg express increased LAG-3, which is required for their maximal suppressive function (Camisaschi C, et al., J. Immunol. 2010; 184:6545-6551 and Huang CT, et al., Immunity. 2004; 21:503-513). Furthermore, ectopic expression of LAG-3 on CD4⁺ effector T cells reduced their proliferative capacity and conferred on them regulatory potential against third party T cells (Huang CT, et al., Immunity. 2004; 21:503-513). Recent studies have also shown that high LAG-3 expression on exhausted lymphocytic choriomeningitis virus (LCMV)-specific CD8⁺ T cells contributes to their unresponsive state and limits CD8⁺ T cell antitumor responses (Blackburn SD, et al., Nat. Immunol. 2009; 10:29-37 and Grosso JF, et al., J. Clin. Invest. 2007; 117:3383-3392). In fact, LAG-3 maintained tolerance to self and tumor antigens via direct effects on CD8⁺ T cells in 2 murine models (Grosso JF, et al., J. Clin. Invest. 2007; 117:3383-3392).

Epstein-Barr virus infection is yet another factor to consider in the potential induction of T cell exhaustion in hematological malignancies. It is known that EBV-associated CLL, Richter's syndrome, and lymphoma cases are usually more aggressive than their EBV(-) counterpart (Tsimberidou AM, et al., Leuk Lymphoma 2006;47:827; Ansell SM, et al., Am J Hematol 1999;60:99.; Dolcetti R, et al., Infectious Agents and Cancer 2010;5:22; Kanakry JA, et al., Blood 2013;121:3547). Interestingly, the expression of checkpoint inhibitors like PD-L1 and LAG-3 has also been documented in EBV-associated malignancies (Green MR, et al., Clin Cancer Res 2012;18:1611; Monti S, et al., Blood 2005;105:1851). High expression of LAG-3 has in fact been documented in chronic viral infections and its blockade with anti-LAG-3 antibodies has been able to reduce viral titers and the expression of checkpoint inhibitors in murine models (Blackburn SD, et al., Nat. Immunol. 2009;10:29-37). Furthermore, LAG-3 expression, alone or in combination with other markers, has been evaluated as a prognostic or predictive marker in CLL and Hodgkin lymphoma (Zhang J, et al., BMC Bioinformatics 2010;11(Suppl 9):S5; Kotaskova J, et al., J Mol Diagn 2010;12(3):328-334). Emerging data indicates that LAG-3 expression on tumor-infiltrating lymphocytes (TILs) and peripheral blood mediates T cell exhaustion in hematological malignancies (Dickinson JD, et al., Leuk Lymphoma 2006;47(2):231-44). Moreover, LAG-3 blockade with specific antibodies has shown antitumor activity in leukemia (Berrien-Elliott, M, et al., Cancer Research 2013; 73(2):605-616) and solid tumor models (Woo, S-R, et al., Cancer Research 2011; 72(4):917-927; Goding, S. R., et al., Journal of Immunology, Baltimore, Md. 1950; 190(9):4899-909). Therefore, LAG-3 is a therapeutic target in hematological malignancies. Its blockade with anti-LAG-3 antibody, alone and in combination with standard of care (e.g., ibrutinib, lenalidomide) or with other checkpoint inhibitors (e.g., nivolumab), deserves further exploration in clinical studies.

WO 2014/008218 describes isolated monoclonal antibodies that specifically bind LAG-3, and their use in treating cancer.

WO 2010/019570 also describes isolated monoclonal antibodies that specifically bind to LAG-3, and their use in treating cancer.

"A Phase 1 Dose Escalation and Cohort Expansion Study of the Safety, Tolerability, and Efficacy of Anti-LAG-3 (BMS-986016) in Relapsed or Refractory Chronic Lymphocytic Leukemia and Lymphomas and Multiple Myeloma" (anonymous; XP055195481; published 2014-11-20) is a clinical study of the safety, tolerability, dose limiting toxicities and maximum tolerated dose of BMS-986016 administered to subjects with relapsed or refractory chronic lymphocytic leukemia, lymphomas and Multiple myeloma.

WO 2015/042246 describes methods for clinical treatment of tumors (e.g., advanced solid tumors) using an anti-LAG-3 antibody in combination with an anti-PD-1 antibody.

Accordingly, it is an object of the present invention to provide improved methods for treating subjects with hematological malignancies using anti-LAG-3 immunotherapy.

### SUMMARY

In one aspect, the invention provides an anti-LAG-3 antibody for use in a method of treating a hematological malignancy in a human patient, as defined in the claims.

The anti-LAG-3 antibodies are for use in methods for treating hematological malignancies (e.g., malignancies derived from myeloid or lymphoid cell lines, such as leukemias, lymphomas, and myelomas) in a human patient, comprising administering to the patient the anti-LAG-3 antibody, wherein the antibody is administered (or is for administration) according to a particular clinical dosage regimen (*i*.*e*., at a particular dose amount and according to a specific dosing schedule). In one embodiment, the human patient suffers from a relapsed or refractory chronic lymphocytic leukemia or lymphoma, such as chronic lymphocytic leukemia (CLL), Hodgkin lymphoma (HL), or non-Hodgkin lymphoma (NHL).

An exemplary anti-LAG-3 antibody is BMS-986016 comprising heavy and light chains having the sequences as set forth in SEQ ID NOs:1 and 2, respectively, or antigen binding fragments and variants thereof (see, e.g., WO 2014/008218). The antibody comprises the heavy and light chain complementarity determining regions (CDRs) or variable regions (VRs) of BMS-986016. The antibody comprises CDR1, CDR2, and CDR3 domains of the heavy chain variable (VH) region of BMS-986016 having the sequence set forth in SEQ ID NO:3, and CDR1, CDR2 and CDR3 domains of the light chain variable (VL) region of BMS-986016 having the sequence set forth in SEQ ID NO:5. The antibody comprises CDR1, CDR2 and CDR3 heavy chain sequences set forth in SEQ ID NOs:7, 8, and 9, respectively, and CDR1, CDR2 and CDR3 light chain sequences set forth in SEQ ID NOs:10, 11, and 12, respectively. In another embodiment, the antibody has VH and/or VL regions comprising the amino acid sequences set forth in SEQ ID NO:3 and/or SEQ ID NO:5, respectively. In another embodiment, the antibody comprises the VH and/or VL regions encoded by the nucleic acid sequences set forth in SEQ ID NO:4 and/or SEQ ID NO:6, respectively. In another embodiment, the antibody competes for binding with, and/or binds to the same epitope on LAG-3 as, the above-mentioned antibodies. In another embodiment, the antibody has at least about 90% variable region amino acid sequence identity with the above-mentioned antibodies (*e*.*g*., at least about 90%, 95% or 99% variable region identity with SEQ ID NO:3 or SEQ ID NO:5).

Accordingly, in one embodiment, antibodies for use in methods for treating a relapsed or refractory chronic lymphocytic leukemia and lymphomas (*e*.*g*., CLL, HL, or NHL) in a human patient are provided, the methods comprising administering to the patient, an effective amount of:
an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,
wherein the method comprises at least one administration cycle, wherein the cycle is a period of eight weeks, wherein for each of the at least one cycles, four doses of the anti-LAG-3 antibody are administered at a dose of 20, 80, 240, or 800 mg.

The anti-LAG-3 antibody is administered at the following doses:
(a) 20 mg anti-LAG-3 antibody;
(b) 80 mg anti-LAG-3 antibody;
(c) 240 mg anti-LAG-3 antibody; or
(d) 800 mg anti-LAG-3 antibody.

The dose of the anti-LAG-3 antibody is a flat-fixed dose.

In another embodiment, the anti-LAG-3 antibody is administered on Days 1, 15, 29, and 43 of each cycle. In another embodiment, the treatment consists of up to 12 cycles.

In one embodiment, the anti-LAG-3 antibody is administered as a first ("front") line of treatment (*e*.*g*., the initial or first treatment). In another embodiment, the anti-LAG-3 antibody is administered as a second line of treatment (*e*.*g*., after initial treatment with the same or a different therapeutic, including after relapse and/or where the first treatment has failed). The anti-LAG-3 antibodies can be administered to a subject by any suitable means. In one embodiment, the antibody is formulated for intravenous administration.

The efficacy of the treatment methods provided herein can be assessed using any suitable means. The treatment produces at least one therapeutic effect, e.g., reduction in the number of malignant cell over time, complete response, partial response, and stable disease.

Also disclosed are kits that include a pharmaceutical composition containing an anti-LAG-3 antibody, such as BMS-986016, and a pharmaceutically-acceptable carrier, in a therapeutically effective amount adapted for use in the methods described herein. In one embodiment, the kit comprises:
(a) a dose of an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5; and
(b) instructions for using the anti-LAG-3 antibody in a method of the invention.

In another aspect, an anti-LAG-3 antibody is provided, the anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, for administration in at least one cycle, wherein for each cycle four doses of the anti-LAG-3 antibody are administered at a dose of 20, 80, 240, or 800 mg.

### BRIEF DESCRIPTION OF THE DRAWINGS

**Figure 1** is a schematic illustrating the parts of a phase I clinical trial.
**Figure 2** is a schematic illustrating the Screening, Treatment, Clinical Follow-up, and Survival Follow-up phases of the clinical trial.
**Figure 3** is a table illustrating the biomarker sampling schedule.
**Figure 4** shows the results of IHC analysis of LAG-3 in NSCLC cells.
**Figure 5** shows the results of IHC analysis of LAG-3 in gastric carcinoma cells.
**Figure 6** is a graph comparing the percentage of LAG-3 positive cells, relative to all the other cell types in the tumor section, in melanoma cells.
**Figure 7** is a graph comparing the percentage of LAG-3 positive cells, relative to all the other cell types in the tumor section, in NSCLC cells.
**Figure 8** is a graph comparing the percentage of LAG-3 positive cells, relative to all the other cell types in the tumor section, in renal cell carcinoma (RCC) cells.
**Figure 9** is a graph comparing the percentage of LAG-3 positive cells, relative to all the other cell types in the tumor section, in gastric carcinoma cells.
**Figure 10** is a graph comparing the percentage of LAG-3 positive cells, relative to all the other cell types in the tumor section, in squamous head and neck carcinoma cells.
**Figure 11** is a table summarizing the percentage of LAG-3 positive cells in lymphoid cells (tumor cells and TILS), relative to all the other cell types in the tumor section, based on LAG-3 light microscopic analysis of non-Hodgkin's lymphoma cells.
**Figure 12** shows the results of IHC analysis of LAG-3 in NHL and DBLCL cells; (A) low power view and (B) high power view.
**Figure 13** shows the results of IHC analysis of LAG-3 in NHL and FL cells; (A) low power view and (B) high power view.
**Figure 14** shows the results of IHC analysis of LAG-3 in NHL, TMA, and CLL cells; (A) low power view and (B) high power view.

### DETAILED DESCRIPTION

### I. Definitions

As used herein, the term "subject" or "patient" is a human cancer patient (*e*.*g*., a patient having an advanced solid tumor, such as an advanced refractory solid tumor).

As used herein, "hematological malignancy" refers to a type of cancer that affects blood, bone marrow, and/or lymph nodes. Such malignancies are characterized by malignant or cancerous cells and are derived from either of the two major blood cell lineages, i.e., the myeloid cell line (which produces granulocytes, erythrocytes, thrombocytes, macrophages and mast cells) or lymphoid cell line (which produces B, T, NK and plasma cells). These cancers include all types of leukemias, lymphomas, and myelomas, e.g., acute, chronic, lymphocytic and/or myelogenous lymphocytic and/or myelogenous leukemias, such as acute lymphocytic leukemia (ALL), acute myelogenous leukemia (AML), chronic lymphocytic leukemia (CLL), and chronic myelogenous leukemia (CML), undifferentiated AML (M0), myeloblastic leukemia (M1), myeloblastic leukemia (M2; with cell maturation), promyelocytic leukemia (M3 or M3 variant [M3V]), myelomonocytic leukemia (M4 or M4 variant with eosinophilia [M4E]), monocytic leukemia (M5), erythroleukemia (M6), megakaryoblastic leukemia (M7), isolated granulocytic sarcoma, and chloroma; lymphomas, such as Hodgkin lymphoma (HL), non-Hodgkin lymphoma (NHL), B-cell lymphomas, T-cell lymphomas, lymphoplasmacytoid lymphoma, monocytoid B-cell lymphoma, mucosa-associated lymphoid tissue (MALT) lymphoma, anaplastic large-cell lymphoma, adult T-cell lymphoma/leukemia, mantle cell lymphoma, angioimmunoblastic T-cell lymphoma, angiocentric lymphoma, intestinal T-cell lymphoma, primary mediastinal B-cell lymphoma, peripheral T- cell lymphoma, lymphoblastic lymphoma, post-transplantation lymphoproliferative disorder, true histiocytic lymphoma, primary central nervous system lymphoma, primary effusion lymphoma, lymphoblastic lymphoma (LBL), Burkitt's lymphoma, diffuse histiocytic lymphoma (DHL), cutaneous T-cell lymphoma (CTLC) (also called mycosis fungoides or Sezary syndrome), and lymphoplasmacytoid lymphoma (LPL) with Waldenstrom's macroglobulinemia; myelomas, such as IgG myeloma, light chain myeloma, nonsecretory myeloma, smoldering myeloma (also called indolent myeloma), solitary plasmocytoma, and multiple myelomas. Particular cancers that may be treated using the antibodies for use in methods of the invention include, e.g., chronic lymphocytic leukemia (CLL), Hodgkin lymphoma (HL), or non-Hodgkin lymphoma (NHL).

As used herein, "effective treatment" refers to treatment producing a beneficial effect, e.g., amelioration of at least one symptom of a disease or disorder. A beneficial effect can take the form of an improvement over baseline, *i*.*e*., an improvement over a measurement or observation made prior to initiation of therapy according to the method. A beneficial effect can also take the form of arresting, slowing, retarding, or stabilizing of a deleterious progression of a marker of a hematological malignancy. Effective treatment may refer to alleviation of at least one symptom of a hematological malignancy. Such effective treatment may, *e*.*g*., reduce patient pain, reduce the size and/or number of malignant cells, may reduce or prevent metastasis of a malignant cell, and/or may slow malignant cell growth.

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In reference to hematological malignancy, an effective amount comprises an amount sufficient to decrease the growth rate of the malignant cells (such as to suppress progression of the malignancy) or to prevent or delay other unwanted malignant cell proliferation. In some embodiments, an effective amount is an amount sufficient to delay malignant cell development. In some embodiments, an effective amount is an amount sufficient to prevent or delay malignant cell recurrence. An effective amount can be administered in one or more administrations. The effective amount of the drug or composition may: (i) reduce the number of malignant cells; (ii) inhibit, retard, slow to some extent and may stop malignant cell infiltration; (iii) inhibit (*i*.*e*., slow to some extent and may stop malignant cell metastasis; (iv) prevent or delay occurrence and/or recurrence of malignant cells; and/or (vii) relieve to some extent one or more of the symptoms associated with the malignancy. In one example, an "effective amount" is the amount of anti-LAG-3 antibody clinically proven to affect a significant decrease in the malignancy or slowing of progression of the malignancy, such as an increase in the number of malignant cells. As used herein, the terms "fixed dose", "flat dose" and "flat-fixed dose" are used interchangeably and refer to a dose that is administered to a patient without regard for the weight or body surface area (BSA) of the patient. The fixed or flat dose is therefore not provided as a mg/kg dose, but rather as an absolute amount of the agent (*e*.*g*., the anti-LAG-3 antibody).

As used herein, a "body surface area (BSA)-based dose" refers to a dose (*e*.*g*., of the anti-LAG-3 antibody) that is adjusted to the body-surface area (BSA) of the individual patient. A BSA-based dose may be provided as mg/kg body weight. Various calculations have been published to arrive at the BSA without direct measurement, the most widely used of which is the Du Bois formula (Du Bois, EF, Arch. Intern. Medicine 1916; 17:863-871; and Verbraecken J, et al., Metabolism ― Clinical and Experimental 2006;55(4): 515-24). Other exemplary BSA formulas include the Mosteller formula (Mosteller, et al., N. Engl. J. Med. 1987; 317:1098), the Haycock formula (Haycock, et al., J. Pediatr. 1978; 93:62-66), the Gehan and George formula (Gehan, et al., Cancer Chemother. Rep. 1970, 54:225-235), the Boyd formula (Current JD, The Internet Journal of Anesthesiology 1998, 2(2); and Boyd, Edith (1935), University of Minnesota. The Institute of Child Welfare, Monograph Series, No. x. London: Oxford University Press), the Fujimoto formula (Fujimoto S, et al., Nippon Eiseigaku Zasshi 1968;5:443-50), the Takahira formula (Fujimoto S, et al., Nippon Eiseigaku Zasshi 1968;5:443-50), and the Schlich formula (Schlich E, et al., Ernährungs Umschau 2010;57:178-183).

The term "antibody" describes polypeptides comprising at least one antibody-derived antigen binding site (e.g., VH/VL region or Fv, or CDR). Antibodies include known forms of antibodies. For example, the antibody can be a human antibody, a humanized antibody, a bispecific antibody, or a chimeric antibody. The antibody also can be a Fab, Fab'2, ScFv, SMIP, Affibody®, nanobody, or a domain antibody. The antibody also can be of any of the following isotypes: IgG1, IgG2, IgG3, IgG4, IgM, IgA1, IgA2, IgAsec, IgD, and IgE. The antibody may be a naturally occurring antibody or may be an antibody that has been altered (*e*.*g*., by mutation, deletion, substitution, conjugation to a non-antibody moiety). For example, an antibody may include one or more variant amino acids (compared to a naturally occurring antibody) which changes a property (*e*.*g*., a functional property) of the antibody. For example, numerous such alterations are known in the art which affect, *e*.*g*., half-life, effector function, and/or immune responses to the antibody in a patient. The term antibody also includes artificial polypeptide constructs which comprise at least one antibody-derived antigen binding site.

The term "LAG-3" refers to Lymphocyte Activation Gene-3. The term "LAG-3" includes variants, isoforms, homologs, orthologs and paralogs. For example, antibodies specific for a human LAG-3 protein may, in certain cases, cross-react with a LAG-3 protein from a species other than human. In other embodiments, the antibodies specific for a human LAG-3 protein may be completely specific for the human LAG-3 protein and may not exhibit species or other types of cross-reactivity, or may cross-react with LAG-3 from certain other species, but not all other species (*e*.*g*., cross-react with monkey LAG-3 but not mouse LAG-3). The term "human LAG-3" refers to human sequence LAG-3, such as the complete amino acid sequence of human LAG-3 having Genbank Accession No. NP_002277 (SEQ ID NO:13). The term "mouse LAG-3" refers to mouse sequence LAG-3, such as the complete amino acid sequence of mouse LAG-3 having Genbank Accession No. NP_032505. LAG-3 is also known in the art as, for example, CD223. The human LAG-3 sequence may differ from human LAG-3 of Genbank Accession No. NP_002277 by having, *e*.*g*., conserved mutations or mutations in non-conserved regions and the LAG-3 has substantially the same biological function as the human LAG-3 of Genbank Accession No. NP_002277. For example, a biological function of human LAG-3 is having an epitope in the extracellular domain of LAG-3 that is specifically bound by an antibody of the instant disclosure or a biological function of human LAG-3 is binding to MHC Class II molecules.

The term "monkey LAG-3" is intended to encompass LAG-3 proteins expressed by Old World and New World monkeys, including but not limited to cynomolgus monkey LAG-3 and rhesus monkey LAG-3. A representative amino acid sequence for monkey LAG-3 is the rhesus monkey LAG-3 amino acid sequence which is also deposited as Genbank Accession No. XM_001108923. Another representative amino acid sequence for monkey LAG-3 is the alternative rhesus monkey sequence of clone pa23-5 as described in US 2011/0150892 A1. This alternative rhesus sequence exhibits a single amino acid difference, at position 419, as compared to the Genbank-deposited sequence.

A particular human LAG-3 sequence will generally be at least 90% identical in amino acid sequence to human LAG-3 of Genbank Accession No. NP_002277 and contains amino acid residues that identify the amino acid sequence as being human when compared to LAG-3 amino acid sequences of other species (*e*.*g*., murine). In certain cases, a human LAG-3 can be at least 95%, or even at least 96%, 97%, 98%, or 99% identical in amino acid sequence to LAG-3 of Genbank Accession No. NP_002277. In certain embodiments, a human LAG-3 sequence will display no more than 10 amino acid differences from the LAG-3 sequence of Genbank Accession No. NP_002277. In certain embodiments, the human LAG-3 can display no more than 5, or even no more than 4, 3, 2, or 1 amino acid difference from the LAG-3 sequence of Genbank Accession No. NP_002277. Percent identity can be determined as described herein.

### II. Anti-LAG-3 Antibodies

The anti-human-LAG-3 antibodies (or VH/VL domains derived therefrom) for use of the invention can be generated using methods well known in the art. Alternatively, art recognized anti-LAG-3 antibodies can be used. For example, the anti-human LAG-3 antibody described in US2011/0150892 A1, and referred to as monoclonal antibody 25F7 (also known as "25F7" and "LAG3.1) can be used. Other art recognized anti-LAG-3 antibodies that can be used include IMP731 described in US 2011/007023.

Antibodies that compete with any of the above-referenced art-recognized antibodies for binding to LAG-3 also can be used.

An exemplary anti-LAG-3 antibody is BMS-986016 comprising heavy and light chains comprising the sequences shown in SEQ ID NOs: 1 and 2, respectively, or antigen binding fragments and variants thereof, as described in WO 2014/008218.

The antibody has the heavy and light chain CDRs or variable regions of BMS-986016. The antibody comprises CDR1, CDR2, and CDR3 domains of the VH region of BMS-986016 having the sequence set forth in SEQ ID NO:3, and CDR1, CDR2 and CDR3 domains of the VL region of BMS-986016 having the sequence set forth in SEQ ID NO:5. The antibody comprises CDR1, CDR2 and CDR3 domains comprising the sequences set forth in SEQ ID NOs:7, 8, and 9, respectively, and CDR1, CDR2 and CDR3 domains comprising the sequences set forth in SEQ ID NOs:10, 11, and 12, respectively. In another embodiment, the antibody comprises VH and/or VL regions comprising the amino acid sequences set forth in SEQ ID NO:3 and/or SEQ ID NO: 5, respectively. In another embodiment, the antibody comprises heavy chain variable (VH) and/or light chain variable (VL) regions encoded by the nucleic acid sequences set forth in SEQ ID NO:4 and/or SEQ ID NO:6, respectively. In another embodiment, the antibody competes for binding with and/or binds to the same epitope on LAG-3 as the above-mentioned antibodies. In another embodiment, the antibody binds an epitope of human LAG-3 comprising the amino acid sequence PGHPLAPG (SEQ ID NO:14). In another embodiment, the antibody binds an epitope of human LAG-3 comprising the amino acid sequence HPAAPSSW (SEQ ID NO:15) or PAAPSSWG (SEQ ID NO:16).

In another embodiment, the antibody has at least about 90% variable region amino acid sequence identity with the above-mentioned antibodies (*e*.*g*., at least about 90%, 95% or 99% variable region identity with SEQ ID NO:3 or SEQ ID NO:5).

### III. Pharmaceutical Compositions

Pharmaceutical compositions suitable for administration to human patients are typically formulated for parenteral administration, *e*.*g*., in a liquid carrier, or suitable for reconstitution into liquid solution or suspension for intravenous administration.

In general, such compositions typically comprise a pharmaceutically acceptable carrier. As used herein, the term "pharmaceutically acceptable" means approved by a government regulatory agency or listed in the U.S. Pharmacopeia or another generally recognized pharmacopeia for use in animals, particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil, glycerol polyethylene glycol ricinoleate, and the like. Water or aqueous solution saline and aqueous dextrose and glycerol solutions may be employed as carriers, particularly for injectable solutions (e.g., comprising an anti-LAG-3 antibody). Liquid compositions for parenteral administration can be formulated for administration by injection or continuous infusion. Routes of administration by injection or infusion include intravenous, intraperitoneal, intramuscular, intrathecal and subcutaneous. In one embodiment, the anti-LAG-3 antibody is administered intravenously.

### IV. Patient Populations

Provided herein are anti-LAG-3 antibodies for use in clinical methods for treating a hematological malignancy (*e*.*g*., a relapsed or refractory chronic lymphocytic leukemia or lymphoma) in human patients.

Examples of cancers that may be treated using the anti-LAG-3 antibodies for use in methods of the invention, include all hematological malignancies derived from either of the two major blood cell lineages, i.e., the myeloid cell line (which produces granulocytes, erythrocytes, thrombocytes, macrophages and mast cells) or lymphoid cell line (which produces B, T, NK and plasma cells). These cancers include all types of luekemias, lymphomas, and myelomas, e.g., acute, chronic, lymphocytic and/or myelogenous leukemias, such as acute lymphocytic leukemia (ALL), acute myelogenous leukemia (AML), chronic lymphocytic leukemia (CLL), and chronic myelogenous leukemia (CML), undifferentiated AML (M0), myeloblastic leukemia (M1), myeloblastic leukemia (M2; with cell maturation), promyelocytic leukemia (M3 or M3 variant [M3V]), myelomonocytic leukemia (M4 or M4 variant with eosinophilia [M4E]), monocytic leukemia (M5), erythroleukemia (M6), megakaryoblastic leukemia (M7), isolated granulocytic sarcoma, and chloroma; lymphomas, such as Hodgkin lymphoma (HL), non-Hodgkin lymphoma (NHL), B-cell lymphomas, T-cell lymphomas, lymphoplasmacytoid lymphoma, monocytoid B-cell lymphoma, mucosa-associated lymphoid tissue (MALT) lymphoma, anaplastic large-cell lymphoma, adult T-cell lymphoma/leukemia, mantle cell lymphoma, angioimmunoblastic T-cell lymphoma, angiocentric lymphoma, intestinal T-cell lymphoma, primary mediastinal B-cell lymphoma, peripheral T- cell lymphoma, lymphoblastic lymphoma, post-transplantation lymphoproliferative disorder, true histiocytic lymphoma, primary central nervous system lymphoma, primary effusion lymphoma, lymphoblastic lymphoma (LBL), Burkitt's lymphoma, diffuse histiocytic lymphoma (DHL), cutaneous T-cell lymphoma (CTLC) (also called mycosis fungoides or Sezary syndrome), and lymphoplasmacytoid lymphoma (LPL) with Waldenstrom's macroglobulinemia; myelomas, such as IgG myeloma, light chain myeloma, nonsecretory myeloma, smoldering myeloma (also called indolent myeloma), solitary plasmocytoma, and multiple myelomas. Particular cancers that may be treated using the anti-LAG-3 antibodies for use in methods of the invention include, e.g., chronic lymphocytic leukemia (CLL), Hodgkin lymphoma (HL), or non-Hodgkin lymphoma (NHL).

In one embodiment, the human patient suffers from a relapsed or refractory chronic lymphocytic leukemia or lymphoma. In a particular embodiment, the human patient suffers from chronic lymphocytic leukemia (CLL), Hodgkin lymphoma (HL), or non-Hodgkin lymphoma (NHL).

Patients can be tested or selected for one or more of the above described clinical attributes prior to, during or after treatment.

### V. Treatment Protocols

Suitable treatment protocols for treating a hematological malignancy in a human patient include, for example, administering to the patient an effective amount of:
an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,
wherein the method comprises at least one administration cycle, wherein the cycle is a period of eight weeks, wherein for each of the at least one cycles, at least four doses of the anti-LAG-3 antibody are administered at a flat dose of about 1, 3, 10, 20, 50, 80, 100, 130, 150, 180, 200, 240, 280, 300, 350, 400, 450, 500, 550, 600, 650, 700, 750, or 800 mg. In another aspect, four doses of the anti-LAG-3 antibody are administered at a dose of 0.01, 0.03, 0.25, 0.1, 0.3, 1 or 3, 5, 8 or 10 mg/kg body weight.

The anti-LAG-3 antibody for use in methods of the invention is administered at the following doses:
(a) 20 mg anti-LAG-3 antibody;
(b) 80 mg anti-LAG-3 antibody;
(c) 240 mg anti-LAG-3 antibody; or
(d) 800 mg anti-LAG-3 antibody.

The dose of the anti-LAG-3 antibody is a flat-fixed dose. In one embodiment, the dose of the anti-LAG-3 antibody is varied over time. For example, the anti-LAG-3 antibody may be initially administered at a high dose and may be lowered over time. In another embodiment, the anti-LAG-3 antibody is initially administered at a low dose and increased over time.

In another embodiment, the amount of the anti-LAG-3 antibody administered is constant for each dose. In another embodiment, the amount of antibody administered varies with each dose. For example, the maintenance (or follow-on) dose of the antibody can be higher or the same as the loading dose which is first administered. In another embodiment, the maintenance dose of the antibody can be lower or the same as the loading dose.

In another embodiment, the anti-LAG-3 antibody is formulated for intravenous administration. In one embodiment, the anti-LAG-3 antibody is administered on Days 1, 15, 29, and 43 of each cycle.

In other embodiments, the anti-LAG-3 antibody is administered once per week, once every or three two weeks, once per month or as long as a clinical benefit is observed or until there is a complete response, confirmed progressive disease or unmanageable toxicity.

A cycle of administration is eight weeks, which can be repeated, as necessary. In another embodiment, the treatment consists of up to 12 cycles.

4 doses of the anti-LAG-3 antibody are administered per eight week cycle.

In another embodiment, the anti-LAG-3 antibody is administered as a first line of treatment (*e*.*g*., the initial or first treatment). In another embodiment, the anti-LAG-3 antibody is administered as a second line of treatment (*e*.*g*., after the initial or first treatment, including after relapse and/or where the first treatment has failed).

### VI. Outcomes

Responses to therapy may include the following criteria:

**2007 INTERNATIONAL WORKING GROUP (IWG) RESPONSE CRITERIA FOR MALIGNANT LYMPHOMA (Cheson, BD, et al. J Clin Oncol. 2007; 25:579)**

| Response | Definition | Nodal masses | Spleen, Liver | Bone marrow |
|---|---|---|---|---|
| CR | Disappearance of all evidence of disease | (a) FDG-avid or PET positive prior to therapy; residual mass of any size permitted if PET negative (b) Variably FDG-avid or PET negative; regression to normal size on CT | Not palpable, nodules disappeared | Infiltrate cleared on repeat biopsy; if indeterminate by morphology, immunohistochemistry should be negative |
| PR | Regression of measurable disease and no new sites | ≥ 50% decrease in SPD of up to 6 largest dominant masses (index lesions); no increase in size of other nodes (non-index lesions) (a) FDG-avid or PET positive prior to therapy; one or more PET positive at previously involved site (b) Variably FDG-avid or PET negative; regression on CT | ≥ 50% decrease in SPD of nodules (for single nodule in greatest transverse diameter); no increase in size of liver or spleen | Irrelevant if positive prior to therapy; cell type should be specified |
| SD | Failure to attain CR/PR or PD | (a) FDG-avid or PET positive prior to therapy; PET positive at prior sites of disease and no new sites on CT or PET (b) Variably FDG-avid or | N/A | N/A |
| | | PET negative; no change in size of previous lesions on CT | | |
| Relapsed disease or PD | Any new lesion or increase by ≥ 50% of previously involved sites from nadir | Appearance of a new lesion(s) > 1.5 cm in any axis, ≥ 50% increase in SPD of more than one node (index lesions), or ≥ 50% increase in longest diameter of a previously identified node > 1 cm in short axis. Lesions PET positive if FDG-avid lymphoma or PET positive prior to therapy | > 50% increase from nadir in the SPD of any previous lesions | New or recurrent involvement |

| | | | | |
|---|---|---|---|---|
| Key: CR = complete remission, CT = computed tomography; FDG = [18F] fluorodeoxyglucose; IWG = International Working Group; NA = Not applicable; PD = progressive disease; PET = positron-emission tomography; PR = partial remission; SD = stable disease; SPD = sum of the product of the diameters. | | | | |

**2008 INTERNATIONAL WORKING GROUP (IWG) RESPONSE CRITERIA FOR CLL WITH MODIFICATIONS (Hallek M, et al., Blood. 2008;111(12):5446 - 5456)**

| **COMPLETE REMISSION (CR)** | | |
|---|---|---|
| • Absence of lymphadenopathy by physical examination and appropriate radiographic techniques. Lymph nodes should not be larger than 1.5 cm in diameter. | | |
| • No hepatomegaly or splenomegaly by physical examination or appropriate radiographic techniques if in a clinical trial. | | |
| • Absence of constitutional symptoms. | | |
| • Normal CBC as exhibited by: | | |
| | - Polymorphonuclear leukocytes ≥ 1,500/µL | |
| | - Blood lymphocytes < 4000/uL | |
| | - Platelets > 100,000/µL | |
| | - Hemoglobin > 11.0 g/dL (without transfusion or exogenous erythropoietin) | |
| • Bone marrow aspirate and biopsy should be performed after clinical and laboratory results demonstrate that all of the requirements listed above have been met, to demonstrate a CR has been achieved. The bone marrow should be analyzed by flow cytometry and/or immunohistochemistry (IHC) to demonstrate that the marrow is free of clonal B-CLL cells. The marrow sample must be at least normocellular for age, with < 30% of nucleated cells being lymphocytes. Lymphoid nodules should assessed by IHC to define whether they are comprised primarily of T cells or lymphocytes other than CLL cells or of CLL cells. Cases with residual CLL cells by conventional flow cytometry or IHC are defined as partial remission (PR). If the bone marrow is hypocellular, a repeat determination should be made in 4 - 6 weeks. Samples should be re-reviewed in conjunction with the prior pathology. | | |
| | | |
| Minimal residual disease (MRD): performed by MRD 4-color flow, allele-specific oligonucleotide PCR with sensitivity to 1 CLL cell per 10,000 leukocytes. | | |

| **COMPLETE REMISSION with incomplete bone marrow recovery (CRi)** Otherwise CR, but who have persistent anemia, thrombocytopenia or neutropenia that appears to be related to persistent drug toxicity rather than to disease activity. The long-term outcome for these patients may be different from the noncytopenic CR. | | |
|---|---|---|
| **PARTIAL REMISSION (PR)** | | |
| | **At least 2 of the following:** | |
| • ≥ 50% decrease in peripheral blood lymphocyte count from the pretreatment baseline. | | |
| • ≥ 50% reduction in the noted pretreatment enlargement of the spleen or liver. | | |
| • 50% reduction in marrow infiltrate, or B-lymphoid nodules. | | |
| • ≥ 50% reduction in lymphadenopathy (preferably by CT) as defined by the following: | | |
| -A decrease in lymph node size by 50% or more either in the sum products of up to 6 lymph nodes, or in the largest diameter of the enlarged lymph node(s) detected prior to therapy. | | |
| -No increase in any lymph node, and no new enlarged lymph node. In small lymph nodes (< 2 cm), an increase of less than 25% is not considered to be significant. | | |
| -A reduction in the noted pretreatment enlargement of the spleen or liver by 50% or more, as detected by CT scan (preferably). | | |
| **At least one of the following:** | | |
| • Polymorphonuclear leukocytes ≥ 1,500/µL or 50% improvement over baseline without need for exogenous growth factors. | | |
| • Platelets > 100,000/ µL or 50% improvement over baseline. | | |
| • Hemoglobin > 11.0g.dL or 50% improvement over baseline without transfusions. | | |
| **PROGRESSIVE DISEASE (PD)** | | |
| **At least one of the following:** | | |
| • Appearance of any new lesion, such as enlarged lymph nodes (> 1.5 cm), de novo splenomegaly, de novo hepatomegaly or other organ infiltrates. | | |
| • An increase by ≥ 50% in greatest determined diameter of any previous site. A lymph node of 1 to 1.5 cm must increase by 50% or more to a size greater than 1.5 cm in the longest axis. A lymph node of more than 1.5 cm must increase to more than 2.0 cm in the longest axis. | | |
| • An increase of 50% or more in the sum of the product of diameters of multiple nodes. | | |
| • ≥ 50% increase in the size of the liver or spleen. | | |
| • ≥ 50% increase in the absolute number of circulating lymphocytes with ≥ 5,000 B lymphocytes per microliter. | | |
| • Transformation to a more aggressive histology (e.g., Richter's syndrome). Whenever possible, this diagnosis should be established by lymph node biopsy. | | |
| • During therapy, cytopenias cannot be used to define disease progression. After treatment the progression of any cytopenia (unrelated to autoimmune cytopenia), as documented by: | | |
| | a. decrease of Hb levels by > 20 g/L (2 g/dL) or to < 100 g/L (10 g/dL), or | |
| | b. decrease of platelet counts by more than 50% or to less than 100 × 109/L (100 000/µL), which occurs at least 3 months after treatment, defines disease progression, if the marrow biopsy demonstrates an infiltrate of clonal CLL cells. | |

| **STABLE DISEASE (SD)** | | |
|---|---|---|
| Subjects who have not achieved a CR or a PR, or who have not exhibited PD. | | |
| **RELAPSE:** defined as a patient who has previously achieved the above criteria ("Complete remission," "Partial remission") of a CR or PR, but after a period of 6 or more months, demonstrate evidence of disease progression (see preceding discussion of progressive disease). | | |

Patients treated according to the methods disclosed herein preferably experience improvement in at least one sign of the malignancy. In one embodiment, improvement is measured by a reduction in the number of malignant cells. In another embodiment, a complete blood count and/or blood film can be used to evaluate responsiveness to a therapy. In another embodiment, a biopsy from a lymph node and/or a bone marrow biopsy can be used to evaluate responsiveness to a therapy.

In one embodiment, the patient treated exhibits a complete response (CR), a partial response (PR), stable disease (SD), immune-related complete disease (irCR), immune-related partial response (irPR), or immune-related stable disease (irSD). In another embodiment, the patient treated experiences a decrease in the growth rate of the malignant cells, *i*.*e*., suppression of malignant cell growth. In another embodiment, recurrence of malignant cells can be prevented or delayed; one or more of the symptoms associated with cancer can be relieved to some extent.

In other embodiments, administration of effective amounts of the anti-LAG-3 antibodies for use in methods of the invention produce at least one therapeutic effect selected from the group consisting of reduction in the number of malignant cells appearing over time, complete remission, partial remission, or stable disease. In still other embodiments, the anti-LAG-3 antibodies for use in methods of the invention produce a comparable clinical benefit rate (CBR = CR+ PR+ SD ≥ 6 months) better than that achieved by an anti-LAG-3 antibody compared to another therapeutic regimen. In other embodiments, the improvement of clinical benefit rate is about 20% 20%, 30%, 40%, 50%, 60%, 70%, 80% or more compared to another therapeutic regimen.

### VII. Kits and Unit Dosage Forms

Also disclosed herein are kits which include a pharmaceutical composition containing an anti-LAG-3 antibody, such as BMS-986016, and a pharmaceutically-acceptable carrier, in a therapeutically effective amount adapted for use in the preceding methods. The kits optionally also can include instructions, *e*.*g*., comprising administration schedules, to allow a practitioner (*e*.*g*., a physician, nurse, or patient) to administer the composition contained therein to administer the composition to a patient having cancer (*e*.*g*., a solid tumor). The kit also can include a syringe.

Optionally, the kits include multiple packages of the single-dose pharmaceutical compositions each containing an effective amount of the anti-LAG-3 for a single administration in accordance with the methods provided above. Instruments or devices necessary for administering the pharmaceutical composition(s) also may be included in the kits. For instance, a kit may provide one or more pre-filled syringes containing an amount of the anti-LAG-3 antibody.

In one embodiment, the present disclosure provides a kit for treating a hematological malignancy in a human patient, the kit comprising:
(a) a dose of an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5; and
(b) instructions for using the anti-LAG-3 antibody in any of the methods and clinical dosage regimens described herein.

The following examples are merely illustrative and should not be construed as limiting the scope of this disclosure in any way as many variations and equivalents will become apparent to those skilled in the art upon reading the present disclosure.

### EXAMPLES

### Example 1: Pre-Clinical Pharamacology of Anti-LAG-3 Antibody (BMS-986016)

### Anti-LAG-3 Antibody. BMS-986016

BMS-986016 is a fully human antibody specific for human LAG-3 that was isolated from immunized transgenic mice expressing human immunoglobulin genes. It is expressed as an IgG4 isotype antibody that includes a stabilizing hinge mutation (S228P) for attenuated Fc receptor binding in order to reduce or eliminate the possibility of antibody- or complement-mediated target cell killing. The heavy and light chain amino acid sequences of BMS-986016 are provided in SEQ ID NOs: 1 and 2, respectively.

The ability of BMS-986016 to bind recombinant human LAG-3 antigen was determined using Biacore and enzyme-linked immunosorbent assay (ELISA). Binding to human and primate LAG-3+ transfectants and to activated human or primate T cells was measured using flow cytometric and Scatchard analyses. BMS-986016 binds to human LAG-3 with high affinity (K_{D} = 0.12-0.5 nM), and inhibits the binding of LAG-3 to cells expressing its ligand, MHC class II (IC50, 0.67 nM). BMS-986016 binds to cynomolgus LAG-3 on transfected CHO cells and on activated cynomolgus T cells with a lower affinity (EC50, 21.5-34.3 nM) than to activated human T cells. A high concentration of BMS-986016, in the absence of secondary co-stimulation, elicits no measurable cytokine response from cultured human peripheral blood cells nor does the drug mediate measurable antibody-dependent or complement-dependent killing of target cells. BMS-986016 promotes the activation of an antigen-specific mouse T cell hybridoma expressing human LAG-3 in co-culture with an MHC class II-positive antigen-presenting cell. In addition, BMS-986016 enhances activation of human T cells in superantigen stimulation assays when added alone or in combination with nivolumab (anti-PD-1 antibody)

### Example 2: Toxicity of Anti-LAG-3 Antibody (BMS-986016)

The following preclinical toxicology studies were performed:

### A. GLP-Compliant Four-Week Intravenous Combination Toxicity Study in Cynomolgus Monkeys with a 6-Week Recovery with BMS-986016 and Nivolumab

The results relating to single-agent treatment with BMS-986016 were as follows:
1. Single-agent BMS-986016 administered at up to 100 mg/kg/week did not result in adverse changes.
2. NOAEL for single-agent BMS-986016 was considered to be 100 mg/kg/week (mean AUC[0-168h] = 474,000 µg·h/mL). The doses administered (100 mg/kg BMS-986016) are ≥ 10 times higher than the maximum doses proposed for the current study.

### B. GLP-Compliant Tissue Cross Reactivity Study in Human and Select Cynomolgus Monkey Tissues with BMS-986016

The results relating to cross reactivity were as follows:
1. Positive staining with BMS-986016-FITC was observed in the plasma membrane or plasma membrane granules following human tissues: mononuclear leukocytes of the urinary bladder, blood cells, colon - large intestine, eye, esophagus, small intestine, stomach, kidney, lung, lymph node, placenta, salivary gland, skin, spleen, thymus, tonsil, uterus - cervix, and uterus - endometrium; and hematopoetic cells of the bone marrow. In addition, staining with BMS-986016-FITC was observed in the cytoplasm of the human pituitary endocrine cell epithelium. Within the limited panel of cynomolgus monkey tissues evaluated, staining with BMS-986016-FITC was observed in the plasma membrane or plasma membrane granules of the mononuclear leukocytes of the spleen.
2. With scientific reports of LAG-3-expressing cells in germinal centers and interfollicular T cell areas of normal human lymphoid tissues (lymph node, tonsil, spleen, thymus, bone marrow and mucosal-associated lymphoid tissue) and having the morphology and distribution of lymphocytes (Huard, et al., Immunogenetics 1994; 39(3):213-217), the staining of mononuclear leukocytes and hematopoietic cells with BMS-986016-FITC in this study (in the human and cynomolgus monkey tissues) was anticipated.
3. Given that LAG-3 mRNA is expressed in the human pituitary and LAG3.1-G4P-FITC staining was observed in adenohypophysis of the human pituitary in a pilot tissue cross reactivity study, BMS-986016-FITC staining of human pituitary endocrine cell epithelium cytoplasm and cytoplasmic granules was also anticipated. Although BMS-986016 is not expected to have access to the cytoplasmic compartment in vivo and the repeat-dose toxicology studies in monkeys showed no effects on the pituitary gland, these findings may be of clinical significance and will be monitored.

### C. In Vitro Cytokine Release and Lymphocyte Activation Assessment with BMS-986016 using Human Peripheral Blood Mononuclear Cells

The results relating to in vitro cytokine release and lymphocyte activation assessment were as follows:
1. BMS-986016 did not induce cytokine release when presented to human peripheral blood mononuclear cells (PBMCs) regardless of concentration, donor, or incubation time. The levels of cytokines observed were either at or near the assay lower limits of quantification with no evidence of dose-dependence or pattern across donors (IL-1β, IL-2, IL-5, IL-10, IL-12p70, and IFN-γ) or were generally overlapping with cytokine levels from PBMCs incubated with negative controls (IL-6, IL-8, TNF-α).
2. Consistent with the lack of cytokine release, there was no evidence that BMS-986016 induced T or NK cell activation, as measured by surface expression of CD25 and CD69. Expression levels of these markers on T and NK cells following stimulation with BMS-986016 were similar to those observed upon stimulation with negative controls.

Overall, these data indicate that BMS-986016 does not possess agonistic potential to induce either T or NK cellular activation or cytokine release.

### Example 3: Preclinical Metabolism and Pharmacokinetics of Anti-LAG-3 Antibody (BMS-986016)

In accordance with regulatory guidelines for biotechnology-derived pharmaceuticals (ICH Harmonised Tripartite Guideline, S6(R1) Preclinical Safety Evaluation of Biotechnology-Derived Pharmaceuticals. International Conference on Harmonisation, 2011), no metabolism studies with BMS-986016 have been conducted in animals. The expected *in vivo* degradation of monoclonal antibodies (mAbs) is to small peptides and amino acids via biochemical pathways that are independent of cytochrome P450 enzymes.

BMS-986016 demonstrated favorable pharmacokinetic (PK) properties in cynomolgus monkeys. From both single-dose and repeat-dose IV PK studies, BMS-986016 decayed bi-exponentially and the exposure was approximately dose-proportional. The systemic clearance (CLTp) ranges from 0.12 to 0.22 mL/h/kg and a terminal half-life (T-HALF) 133 to 414 hours. The volume of distribution at steady state (Vss) was 62 to 72 mL/kg, suggesting limited distribution outside the plasma. Anti-BMS-986016 antibodies were detected in some monkeys but the presence of anti-BMS-986016 antibodies appeared to have no impact on BMS-986016 exposure.

### Example 4: Phase 1 Trial in Patients Having Relapsed or Refractory CLL and Lymphomas

A phase 1 trial of anti-LAG-3 antibody (BMS-986016) is conducted in patients having relapsed or refractory CLL and lymphomas to demonstrate the efficacy of administering BMS-986016 as a treatment.

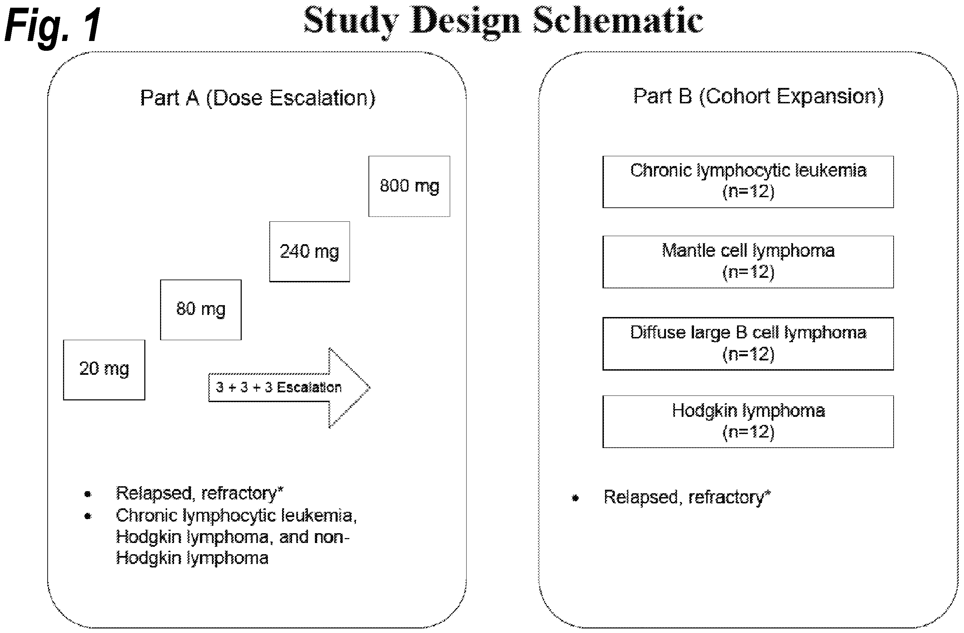

This is a Phase 1, open-label study of BMS-986016 administered as a single agent to subjects with relapsed or refractory CLL and lymphomas. The study will be conducted in 2 parts. Part A consists of a 3 + 3 + 3 dose escalation design in subjects with relapsed or refractory CLL, HL, and NHL. Part B consists of cohort expansion in 4 disease-restricted populations of approximately 12 subjects each (Figure 1). Treatment in Part B will be initiated when the MTD (or MAD if no MTD is established) for Part A has been determined.

Subjects will complete up to 3 periods of the study: Screening (up to 28 days), Treatment (up to a maximum of twelve 8-week cycles of study therapy), and Clinical Follow-up (135 days following the last dose of study drug; a longer follow-up period could be considered in selected cases if an efficacy signal is apparent). WOCBP will have additional follow-up assessments through Day 165 for home pregnancy tests.

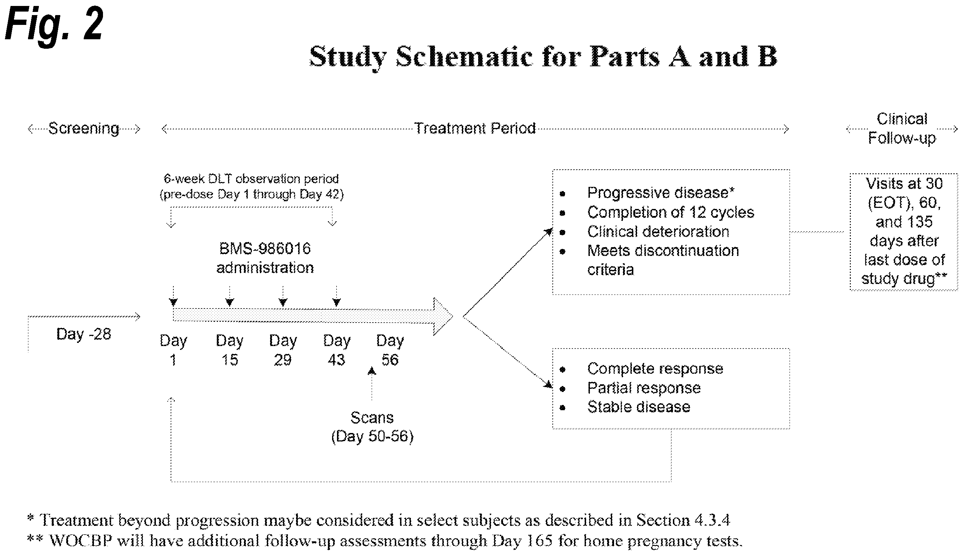

The Treatment Period consists of up to twelve 8-week treatment cycles. Each treatment cycle comprises 4 doses of BMS-986016 administered on Days 1, 15, 29, and 43. Subjects will be allowed to continue study therapy until the first occurrence of either: (1) meeting criteria for discontinuation, (2) completion of the maximum number of twelve 8-week cycles, (3) confirmed progressive disease (PD), or (4) clinical deterioration. Subjects who discontinue treatment will enter a 135-day Clinical Follow-up period (Figure 2).

Physical examinations, vital sign measurements, 12-lead electrocardiograms (ECG), pulse oximetry, and clinical laboratory evaluations will be performed at selected times throughout the dosing interval. Subjects will be closely monitored for AEs throughout the study. Blood will be collected following the start of study drug administration for pharmacokinetic (PK) analysis.

Subjects will be allowed to continue on therapy for up to twelve 8-week cycles, confirmed PD, or until meeting criteria for discontinuation as described in herein. Subjects may be on study for a total of up to approximately 2.3 years, including a 28-day screening period, up to twelve 8-week cycles of treatment, and a 135-day clinical follow-up period. The total duration of the study is expected to be approximately 4.3 years from the time of the first visit of the first subject to the required follow-up of the last subject enrolled.

### Part A: Dose Escalation

In Part A, a 3 + 3 + 3 design will be used to assess the safety of BMS-986016. The dose levels evaluated during dose escalation are provided in Figure 1 and Table 1 (set froth below). Three subjects will initially be treated in each dose cohort; in Dose Cohort 1, the first 3 subjects will be designated as sentinel subjects and will begin treatment at least 5 days apart. Subjects in subsequent cohorts will not be required to observe the 5-day interval between treatment start dates.

Dose escalation will be based on the number of DLTs experienced during the DLT evaluation interval as determined by the Medical Monitor and Investigators. The DLT evaluation interval begins on the first day of treatment and continues for 8 weeks, ie, through Day 56 of the first cycle.

Dose escalation in Part A will proceed as follows:
- If none of the first 3 evaluable subjects in a dose cohort experiences a DLT within the DLT evaluation interval, then the next 3 subjects will be treated at the next higher dose cohort.
- If 1 of the first 3 evaluable subjects in a cohort experiences a DLT within the DLT evaluation interval, then 3 additional subjects will be treated in that dose cohort.
- If no more than 1 of the first 6 evaluable subjects experiences a DLT during the DLT evaluation interval, then the next 3 subjects will be enrolled at the next higher dose cohort.
- If 2 of the first 6 evaluable subjects in a cohort experience a DLT, that cohort will be expanded to 9 evaluable subjects.
- If ≥ 2 of the first 3 evaluable subjects, ≥ 3 of the first 6 evaluable subjects, or ≥ 3 of the first 9 evaluable subjects in a cohort experience a DLT within the DLT evaluation interval, that dose level will have exceeded the MTD and dose escalation will be terminated.

**Table 1: Dose Escalation Schedule for Part A**

| **Dose Cohort Number** | **BMS-986016 Dose (IV; mg)** | | **Total Subjects** |
|---|---|---|---|
| 1 | 20 | | n = approximately 3 to 9 |
| 2 | 80 | | n = approximately 3 to 9 |
| 3 | 240 | | n = approximately 3 to 9 |
| 4 | 800 | | n = approximately 3 to 9 |
| Total | | N = approximately 12 to 36 | |

### Part B: Cohort Escalation

The purpose of cohort expansion is to gather additional safety, tolerability, preliminary efficacy, PK, and pharmacodynamic information regarding BMS-986016. The dose selected for Part B will not exceed the MTD (or MAD if MTD is not determined) in Part A, may be a dose intermediate to the doses evaluated in Part A, and may incorporate assessment of other data including delayed toxicities and PK and pharmacodynamic data from Part A. Modeling may also be used to help inform the selection of the dose evaluated in Part B.

Four expansion cohorts will be restricted to the tumor types listed in Figure 1 and Table 2 (set forth below). Continuous evaluation of toxicity events will be assessed throughout enrollment in the expansion cohorts. If, at any time, the aggregate rate of treatment-related toxicities meeting DLT criteria exceeds 33% across all subjects treated in the Part B cohort expansions, the findings will be discussed with the Medical Monitor and Investigators; further enrollment may be interrupted. Depending on the nature and grade of the toxicity and after assessing the risk:benefit ratio, the dose for all or for select cohorts may be reduced.

**Table 2: Tumor Types Eligible For Part B ― Cohort Expansion**

| **Tumor Type^{a}** | **Total Subjects** |
|---|---|
| Chronic Lymphocytic Leukemia (CLL) | approximately 12 |
| Diffuse Large B-Cell Lymphoma (DLBCL) | approximately 12 |
| Mantle Cell Lymphoma (MCL) | approximately 12 |
| Hodgkin Lymphoma (HL) | approximately 12 |
| Total | approximately 48 |

| | |
|---|---|
| ^{a} All subjects in Part B will be naive to immune cell-modulating antibody regimens (ICMARs), such as, but not limited to anti- CTLA-4 , anti-PD-1, anti―PD-L1, anti-PD-L2, anti-KIR, anti-CD137, and/or anti-OX40 antibodies except for anti-CD20, alemtuzumab, or brentuximab antibody therapy. | |

### Dose-Limiting Toxicities

For the purpose of guiding decisions regarding dose escalation in Part A, dose-limiting toxicity (DLT) will be determined based on the incidence, intensity, and duration of adverse events (AEs) that are considered related to study treatment. The DLT evaluation interval begins on the first day of treatment and continues through Day 56 of the first cycle (i.e., 8 weeks). Adverse events will be graded according to National Cancer Institute (NCI) Common Terminology Criteria for Adverse Events (CTCAE) v4.0. For the purpose of subject management, any AE that meets DLT criteria regardless of the cycle in which it occurs, will lead to dose interruption.

Dose escalation will be based on the number of DLTs experienced during the DLT evaluation interval as determined by the Medical Monitor and Investigators. No intrasubject dose escalation is allowed. Subjects who receive at least 1 dose of study drug during the 8-week evaluation interval will be considered evaluable for DLT determination. Subjects who withdraw from the study during the DLT evaluation interval for reasons other than a DLT and/or for whom safety data are unavailable for the entire DLT evaluation interval may be replaced at the same dose level. In the event that an infusion cannot be administered at a scheduled visit during the DLT evaluation interval, it must be administered as soon as possible. If the delay is between 1 and 7 days, the procedures at the originally scheduled visit should be performed and subjects will be considered evaluable for DLT determination. If the delay is more than 7 days, the dose will be considered missed and will not be replaced. Subjects with a delay of more than 7 days will not be considered evaluable for DLT determination. Unevaluable subjects may be replaced at the same dose level.

### Duration of Study

Subjects will be allowed to continue on therapy for up to twelve 8-week cycles, confirmed PD, or until meeting criteria for discontinuation. Subjects may be on study for a total of up to approximately 2.3 years, including a 28-day screening period, up to twelve 8-week cycles of treatment, a 135-day clinical follow-up period, and for WOCBP, additional home pregnancy tests through Day 165. The total duration of the study is expected to be approximately 4.3 years from the time of the first visit of the first subject to the required follow-up of the last subject enrolled.

### Number of Subjects

Approximately 84 subjects may be dosed (approximately 36 subjects during dose escalation and up to 48 subjects in cohort expansion).

### Study Population

Male and female subjects with histologic or cytologic confirmation of CLL, Hodgkin lymphoma, or non-Hodgkin lymphoma (including T-cell and B-cell lymphomas) who have relapsed following prior treatment or been refractory to prior treatment and who meet all entry criteria will be eligible to participate.

### Dose Escalation (Part A)

Adult subjects with relapsed or refractory non-Hodgkin lymphoma (NHL), Hodgkin lymphoma (HL), or chronic lymphocytic leukemia (CLL) are eligible.

### Cohort Expansion (Part B)

Four disease-restricted groups will be permitted during cohort expansion per Table 2 (above).

### Parts A and B

Neutrophil count must be > 750/µL and platelet count > 50,000/µL. Subjects with primary cutaneous lymphoma, lymphoproliferative diseases associated with primary immune deficiencies, and lymphomas associated with human immunodeficiency virus (HIV) infection are excluded. Subjects with autoimmune disorders are also excluded.

Women must not be nursing or pregnant. WOCBP must have a negative pregnancy test within 24 hours prior to receiving their first dose of study medication. WOCBP must agree to follow instructions for method(s) of contraception for a total of 24 weeks after their last dose of investigational drug (a period of 30 days plus the time required for the investigational drug to undergo 5 half-lives (i.e., 165 days total or 24 weeks).

Men who are sexually active with WOCBP must agree to follow instructions for method(s) of contraception based on the information in Appendix 3 for a total of 33 weeks after their last dose of investigational drug (a period of 90 days plus the time required for the investigational drug to undergo 5 half-lives (i.e., 225 days total or 33 weeks).

Hepatic, non-hematologic, and hematologic DLTs are defined separately as outlined below.

### 1. Hepatic DLT

Any of the following drug-related events will be considered a hepatic DLT:
- Alanine aminotransferase (ALT) or aspartate aminotransferase (AST) > 8 × ULN, regardless of duration
- ALT or AST > 5 × and ≤ 8 × ULN, that fails to return to ≤ Grade 1 within 2 weeks despite medical intervention
- Total bilirubin > 5 × ULN
- ALT or AST > 3 × ULN and concurrent total bilirubin > 2 × ULN

### 2. Non-Hematologic DLT

Any of the following drug-related events will be considered a non-hematologic DLT:
- Grade 2 eye pain or reduction in visual acuity that requires systemic treatment
- Grade 2 eye pain or reduction in visual acuity that does not respond to topical therapy and that does not improve to Grade 1 within 2 weeks of initiation of topical therapy
- ≥ Grade 3 non-hepatic or non-hematologic toxicity with the exceptions noted below The following Grade 3 non-hematologic events will not be considered DLTs:
- Grade 3 electrolyte abnormality that lasts < 72 hours, is not clinically complicated, and resolves spontaneously or responds to conventional medical intervention
- Grade 3 increase in amylase or lipase that is not associated with symptoms, clinical manifestations, or radiographic evidence of pancreatitis
- Grade 3 nausea or vomiting that lasts < 48 hours and resolves to ≤ Grade 1 either spontaneously or with conventional medical intervention
- Grade 3 fever that resolves within 72 hours and is not associated with hemodynamic compromise (including hypotension, or clinical or laboratory evidence of end organ perfusion impairment)
- Grade 3 tumor flare (defined as pain, irritation, or rash that localizes to sites of known or suspected tumor)
- Grade 3 fatigue for less than 7 days

### 3. Definition of Hematologic DLT

Any of the following drug-related events will be considered a hematologic DLT:
- Grade 4 anemia
- Grade 4 febrile neutropenia of any duration
- Grade 4 neutropenia that does not resolve to Grade 3 or less within 5 days of initiation of granulocyte colony stimulating factor (G-CSF)
- Platelet transfusion or a platelet count < 10,000/µI
- Grade 3 thrombocytopenia associated with clinically significant bleeding
- Grade 3 hemolysis
- Grade 3 anemia in subjects with ≤ Grade 1 anemia at baseline

Subjects who receive at least 1 dose of study drug during the 8-week evaluation interval will be considered evaluable for DLT determination. In the event that study drug cannot be administered at a scheduled visit during the DLT evaluation interval, it must be administered as soon as possible. If the delay is between 1 and 7 days, the procedures at the originally scheduled visit should be performed and subjects will be considered evaluable for DLT determination. If the delay is more than 7 days, the dose will be considered missed and will not be replaced. Subjects with a delay of more than 7 days will not be considered evaluable for DLT determination. Unevaluable subjects may be replaced at the same dose level. Subjects who miss a dose during the DLT evaluation period may continue on treatment if the subject does not otherwise meet the criteria for permanent discontinuation.

### Inclusion Criteria

### 1. Signed Written Informed Consent

The subject must sign and date the IRB/IEC-approved written informed consent form prior to the performance of any study-related procedures that are not considered part of standard of care.

Consent for biopsy samples:
(i) Subjects must consent to allow a pre-treatment tumor biopsy (e.g., lymph node) to be performed (all subjects). If a pre-treatment tumor biopsy is not clinically feasible, subject must consent to allow the acquisition of an archived tumor sample (e.g., primary tumor, lymph node, etc.). Subjects unable to provide a fresh pre-treatment tumor biopsy or archived tumor sample are not eligible. Subjects whose pre-treatment biopsy yields inadequate tissue quantity or quality will not be ineligible on this basis alone.
(ii) Subjects must consent to allow a pre-treatment unilateral bone marrow biopsy and/or aspirate to be performed (all subjects) and on treatment at complete response (CR), partial response (PR), or progressive disease (PD), as clinically indicated. Subjects who had a bone marrow biopsy and/or aspirate since completion of their last therapy may not use those results in lieu of the required baseline bone marrow biopsy.

### 2. Target Population

(a) Subjects must have histologic or cytologic confirmation of chronic lymphocytic leukemia, Hodgkin lymphoma, or Non-Hodgkin lymphoma and have relapsed following prior treatment or been refractory to prior treatment.
(b) Part A Dose Escalation:
   (i) Chronic lymphocytic lymphoma
   (ii) Hodgkin lymphoma
   (iii) Non-Hodgkin lymphoma
(c) Part B Cohort Expansion
   (i) Chronic lymphocytic leukemia
   (ii) Diffuse large B-cell lymphoma
   (iii) Mantle cell lymphoma
   (iv) Hodgkin lymphoma
   (v) Multiple myeloma
(d) Subjects must have at least one measureable lesion > 1.5 cm as defined by the Lymphoma (Cheson et al., J. Clin. Oncol. 2007;25(5):579-586) and CLL (Hallek et al., Blood 2008;111(12)5446-5456) response criteria. Tumor sites that are considered measureable must not have received prior radiation therapy
(e) Only subjects without prior exposure to immune cell-modulating antibody regimens (ICMARs), such as anti-CTLA-4, anti-PD-1, anti―PD-L1, anti-PD-L2, anti-KIR, anti- CD137, or anti-OX40 antibodies, are allowed. Prior anti-CD20, alemtuzumab, or brentuximab antibody therapy is allowed.
(f) Subjects must have progressed or be refractory to, at least one prior standard therapy, including radiation, immunotherapy, cytotoxic chemotherapy, and select antibody (anti- CD20, alemtuzumab, or brentuximab) therapy. The following are not considered separate lines of treatment: addition of a compound to an ongoing regimen, restarting the same regimen after a drug holiday, or switching from IV to oral therapy.
(g) Subjects are not eligible for transplantation or any standard therapy known to be life prolonging or life-saving. (Subjects who are eligible for transplantation or any standard therapy known to be life-prolonging or life-saving and who have declined transplantation or any standard therapy known to be life-prolonging or life-saving are eligible for the study.
(h) Subjects must be more than 100 days post autologous transplant
(i) Eastern Cooperative Oncology Group (ECOG) status of 0 or 1
(j) Life expectancy of ≥1 0 weeks at the time of informed consent per Investigator assessment
(k) Adequate organ function as defined by the following:
   (i) Neutrophils ≥ 500/µL (stable off any growth factor within 1week of first study drug administration)
   (ii) Platelets ≥ 50 × 103/µL (transfusion to achieve this level is not permitted within 2 weeks of first study drug administration)
   (iii) Hemoglobin ≥ 8.5 g/dL (transfusion to achieve this level is not permitted within 2 weeks of first study drug administration)
   (iv) Creatinine < 1.5 × ULN or creatinine clearance ≥ 40 mL/min (Cockcroft-Gault formula)
   (v) ALT and AST ≤ 3 × ULN
   (vi) Total bilirubin ≤ 1.5 × ULN (except subjects with Gilbert's syndrome who must have normal direct bilirubin)
   (vii) Normal thyroid function, or stable on hormone supplementation per Investigator assessment
(l) Ability to comply with treatment, PK, and pharmacodynamic sample collection and required study follow-up.
(m) Subject re-enrollment: This study permits the re-enrollment of a subject that has discontinued the study as a pre-treatment failure (ie, subject has not been randomized or treated). If re-enrolled, the subject must be re-consented.

### 3. Age and Reproductive Status

(a) Men and women, ages ≥ 18 years at the time of informed consent
(b) Women of childbearing potential (WOCBP) must have a negative serum or urine pregnancy test (urine pregnancy test: minimum sensitivity 25 IU/L or equivalent units of human chorionic gonadotropin [hCG]) within 24 hours prior to the start of study drug.
(c) Women must not be breastfeeding.
(d) WOCBP must agree to follow instructions for method(s) of contraception for the duration of treatment with BMS-986016 plus 5 half-lives of BMS-986016 (135 days) plus 30 days (duration of ovulatory cycle) for a total of 165 days (24 weeks) after completion of treatment.
(e) Men who are sexually active with WOCBP must agree to follow instructions for method(s) of contraception for the duration of treatment with BMS-986016 plus 5 half-lives of BMS-986016 (135 days) plus 90 days (duration of sperm turnover) for a total of 225 days (33 weeks) after completion of treatment.

Investigators shall counsel WOCBP and male subjects who are sexually active with WOCBP on the importance of pregnancy prevention and the implications of an unexpected pregnancy. Investigators shall advise WOCBP and male subjects who are sexually active with WOCBP on the use of highly effective methods of contraception. Highly effective methods of contraception have a failure rate of < 1% per year when used consistently and correctly.

At a minimum, subjects must agree to the use of 2 methods of contraception, with one method being highly effective and the other being either highly effective or less effective.

(f) Azoospermic males and WOCBP who are continuously not heterosexually active are exempt from contraceptive requirements. However, WOCBP who abstain from heterosexual activity on a continuous basis must still undergo pregnancy testing as described in this protocol

### Exclusion Criteria

### 1. Target Disease Exceptions

(a) Subjects with primary cutaneous lymphoma, lymphoproliferative diseases associated with primary immune deficiencies, and lymphomas associated with human immunodeficiency virus (HIV) infection are excluded.
(b) Subjects with known or suspected central nervous system (CNS) metastases or with the CNS as the only site of active disease are excluded:
   (i) Subjects with controlled brain metastases will be allowed to enroll. Controlled brain metastases are defined as those with no radiographic progression for at least 4 weeks after radiation or surgical treatment at the time of consent. Subjects must have been off steroids for at least 2 weeks prior to informed consent and have no new or progressive neurological signs and symptoms.
   (ii) Subjects with signs or symptoms of brain metastases are not eligible unless brain metastases are ruled out by computed tomography (CT) or magnetic resonance imaging (MRI).

### 2. Medical History and Concurrent Diseases

(a) Subjects with a prior malignancy are excluded, except adequately treated basal cell or squamous cell skin cancer, carcinoma in situ of the cervix or of the bladder, or in situ ductal or lobular carcinoma of the breast. Subjects with other prior malignancies diagnosed more than 2 years previously (at the time of informed consent) who have received therapy with curative intent with no evidence of disease during the interval and who are considered by the Investigator to present a low risk for recurrence will be eligible.
(b) Subjects with any active autoimmune disease or history of known or suspected autoimmune disease with the exception of subjects with isolated vitiligo, resolved childhood asthma/atopy, controlled hypoadrenalism or hypopituitarism, and euthyroid patients with a history of Grave's disease (subjects with controlled hyperthyroidism must be negative for thyroglobulin and thyroid peroxidase antibodies and thyroid-stimulating immunoglobulin prior to study drug administration).
(c) Subject has autoimmune hemolytic anemia (AIHA) or autoimmune thrombocytopenia (ITP) requiring therapeutic doses of systemic steroids
(d) Subject has undergone any allogeneic transplant
(e) A known or underlying medical condition that, in the opinion of the Investigator or Sponsor, could make the administration of study drug hazardous to the subject or could adversely affect the ability of the subject to comply with or tolerate study procedures and/or study therapy, or confound the ability to interpret the tolerability of BMS-986016 in treated subjects
(f) Requirement for daily supplemental oxygen
(g) Uncontrolled or significant cardiovascular disease including, but not limited to, any of the following:
   (i) Myocardial infarction or stroke/transient ischemic attack (TIA) within the 6 months prior to consent
   (ii) Uncontrolled angina within the 3 months prior to consent
   (iii) Any history of clinically significant arrhythmias (such as ventricular tachycardia, ventricular fibrillation, or torsades de pointes)
   iv) QTc prolongation > 480 msec
   (v) History of other clinically significant heart disease (i.e., cardiomyopathy, congestive heart failure with New York Heart Association (NYHA) functional classification III-IV, pericarditis, significant pericardial effusion)
(h) Positive blood screen for HIV or known acquired immunodeficiency syndrome (AIDS)
(i) History of any chronic hepatitis as evidenced by:
   (i) Positive test for hepatitis A antibody (HepA IgM). Note: history of resolved hepatitis A virus infection is not an exclusion criterion.
   (ii) Positive test for hepatitis B surface antigen (HBsAg) and/or hepatitis B core antigen
   (iii) Positive test for qualitative hepatitis C viral load (by PCR)
(j) Evidence of active infection that requires systemic antibacterial, antiviral, or antifungal therapy ≤ 7 days prior to initiation of study drug therapy
(k) Any other significant acute or chronic medical illness
(l) Subjects who are unable to undergo venipuncture and/or tolerate venous access
(m) Any other sound medical, psychiatric, and/or social reason as determined by the Investigator.
(n) Any of the following procedures or medications:
   (i) Within 2 weeks prior to time of informed consent:
      (1) Systemic or topical corticosteroids at immunosuppressive doses (≥ 7.5 mg/day of prednisone or equivalent)
      (2) Medicinal herbal preparations
   (ii) Within 4 weeks prior to study drug administration:
      (1) Any investigational drug or placebo
      (2) Any anticancer therapy (chemotherapy, monoclonal antibody for antineoplastic intent [e.g., anti-CD20, anti-CD30, or alemtuzumab antibody therapy], therapeutic vaccines, radiotherapy, or hormonal treatment)
      (3) Non-oncology vaccines containing live virus
      (4) Allergen hyposensitization therapy
      (5) Major surgery
   (iii) Within 6 weeks prior to study drug administration:
      (1) Nitrosureas, fludarabine
   (iv) Within 10 weeks prior to study drug administration: (1) Radio- or toxin-immunoconjugates (eg, brentuximab)

### 3. Allergies and Adverse Drug Reaction

(a) History of allergy to components of BMS-986016 (e.g., history of severe hypersensitivity reactions to drugs formulated with polysorbate 80)

### 4. Other Exclusion Criteria

(a) Prisoners or subjects who are involuntarily incarcerated
(b) Subjects who are compulsorily detained for treatment of either a psychiatric or physical (e.g., infectious disease) illness
(c) Inability to comply with restrictions and prohibited activities/treatments

### Safety Assessments

Adverse events are assessed continuously during the study and for 135 days after the last treatment. Adverse events are evaluated according to the NCI CTCAE version 4.0. Adverse events are coded using the most current version of Medical Dictionary for Regulatory Activities (MedDRA) and reviewed for potential significance and importance.

### Efficacy Assessments

Efficacy assessments will be conducted and reported on the eCRF using the appropriate efficacy assessment based on tumor type. Subjects with NHL or HL will be evaluated using the Revised Response Criteria for Malignant Lymphoma (Cheson et al., J. Clin. Oncol. 2007;25(5):579-586). Subjects with CLL will be evaluated using the Guidelines for the Diagnosis and Treatment of Chronic Lymphocytic Leukemia (Hallek et al., Blood 2008;111(12):5446-56).

### Secondary Efficacy Assessments

Disease assessments will occur between Days 50 and 56 of each treatment cycle (up to twelve 8- week treatment cycles) and at the 30-day follow-up visit. Tumor responses will be evaluated by the investigator for subjects with adequate data as defined by the following efficacy criteria:
- NHL and HL subjects: Revised Response Criteria for Malignant Lymphoma
- CLL subjects: Guidelines for the Diagnosis and Treatment of Chronic Lymphocytic Leukemia

### Pharmacokinetic Sample Analyses

The serum samples will be analyzed for BMS-986016 by a validated immunoassay. In addition, selected serum samples may be analyzed by an exploratory analytical method that measures BMS-986016 for technology exploration purposes; exploratory data will not be reported.

### Exploratory Biomarker Assessments

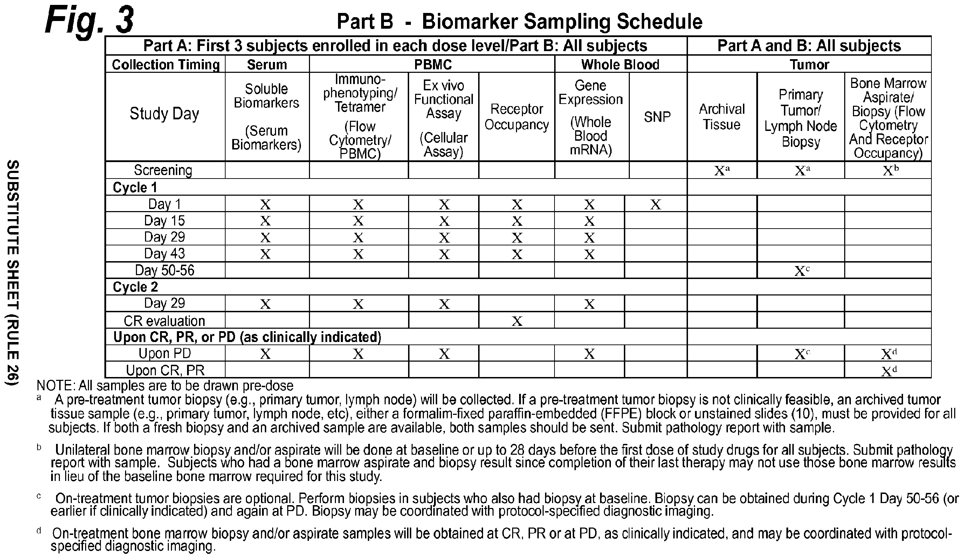

Tumor tissue, bone marrow, and/or aspirate will be collected prior to therapy and at selected timepoints on treatment in all subjects in Parts A and B. Peripheral blood will be collected prior to therapy and at selected timepoints on treatment in the first 3 subjects enrolled in each dose level in Part A and in all subjects in Part B. If biomarker samples are drawn but study drug is not administered, samples will be retained. A schedule of pharmacodynamic evaluations is provided in Figure 3.

### Soluble Biomarkers

Soluble factors, such as cytokines, chemokines, soluble receptors, and antibodies to tumor antigens will be characterized and quantified by immunoassays in serum. Analyses may include, but not necessarily be limited to, soluble CD25, soluble PD-1, soluble LAG-3, and CXCL-9. Collected serum samples will also be used for the assessment of tumor antigen-specific responses elicited following treatment with monotherapy to explore which antitumor antibodies are most associated with clinical response. Antibody levels to cancer test antigens will be assessed by multiplex assays and ELISA.

### Immunophenotyping of PBMC and Bone Marrow Aspirates

The proportion of specific lymphocyte subsets and expression levels of T-cell co-stimulatory markers in PBMC preparations will be quantified by flow cytometry. Analyses may include, but not necessarily be limited to, the proportion of T, B, and NK cells, proportion of memory and effector T cell subsets, and expression levels of LAG-3, PD-1, PD-L1, PD-L2, ICOS, and Ki67.

### Ex Vivo Functional Assays

To explore whether nivolumab will restore T cell activation and function, PBMCs will be isolated and cryopreserved. Assays of the functional status of effector T cells will be performed, including but not limited to, assays for interferon-gamma (IFN-γ) and CD107.

### Peripheral Blood Gene Expression

The expression level of genes related to response to BMS-986016 will be quantified using molecular methods such as Affymetrix microarray and/or quantitative RT-PCR analysis in whole blood samples. Analysis may include, but not necessarily be limited to, genes associated with immune-related pathways, such as T cell activation and antigen processing and presentation.

### BMS-986016 Receptor Occupancy

The percentage of BMS-986016 bound to immune cells, as well as mean fluorescent intensity (MFI) of bound drug, will be quantified by a flow cytometry-based receptor occupancy assay. This assay will be performed on peripheral blood and, where available, bone marrow aspirates. The receptor occupancy assay will provide data to determine the amount and type of T cells that the drug is interacting with in different biological compartments. Samples will be taken prior to treatment to define a baseline and at the time of bone marrow aspirate collection. Analysis of peripheral blood and bone marrow aspirates will be done in fresh samples; therefore, it is critical that samples be shipped promptly after collection.

### T-Cell Repertoire Analysis

Low diversity of the peripheral T-cell compartment has been shown to correlate with poor overall survival in metastatic breast cancer (Manuel et al., Oncoimmunology 2012;1(4):432-440. A standing theory in immuno-oncology suggests a diverse and activated immune environment is better adept at eradicating tumor compared to a skewed repertoire of naive and tolerized T cells. In order to explore whether a diverse T-cell repertoire is predictive of response to therapy, next generation, high-throughput DNA sequencing will be performed on DNA isolated from peripheral blood and tumor tissue to quantitate the composition of the T-cell repertoire prior to, and during, monotherapy.

### SNP Analysis

In order to identify potential polymorphisms associated with safety and efficacy of BMS-986016, selected genes will be evaluated for single nucleotide polymorphisms (SNP). Analysis will be limited to sequence polymorphisms linked to genes and pathways associated with PD-1/PD-L1, LAG-3, and activated T-cell phenotype, including PD-1, PD-L1, PD-L2, LAG-3, MHC II, and CTLA-4.

### Tumor-Based Biomarker Measures

Tumor biopsy specimens (e.g., primary tumor, lymph nodes) will be obtained prior to and after treatment with BMS-986016 to characterize immune cell populations and expression of selected tumor markers.

A pre-treatment tumor biopsy (e.g., primary tumor, lymph node) will be collected in all consenting adults. If a pre-treatment tumor biopsy is not clinically feasible, an archived tumor tissue sample (e.g., primary tumor, lymph node, etc.), either a formalin-fixed paraffin-embedded (FFPE) block or unstained slides, must be provided for performance of correlative studies. The pathology report should be submitted with the archived or fresh biopsy sample. If both a fresh biopsy and an archived sample are available, both samples should be sent.

On-treatment tumor biopsies are optional and will be collected in subjects who had a biopsy at baseline. The biopsy can be obtained during Cycle 1 Days 50 to 56 (or earlier if clinically indicated) and again at PD. The biopsy may be coordinated with protocol-specified diagnostic imaging.

Unilateral bone marrow biopsy and/or aspirate will be done at baseline or up to 28 days before the first dose of study drug on all subjects. Subjects who had a bone marrow aspirate and biopsy result since completion of their last therapy may not use those bone marrow results in lieu of the baseline bone marrow required for this study. On-treatment bone marrow biopsy and/or aspirate samples will be obtained at CR, PR or at PD, as clinically indicated, and may be coordinated with protocol-specified diagnostic imaging.

Biopsy and bone marrow samples may be used for the following assessments:

### Characterization of tumor infiltrating lymphocytes (TILs), tumor antigens, and viral positivity

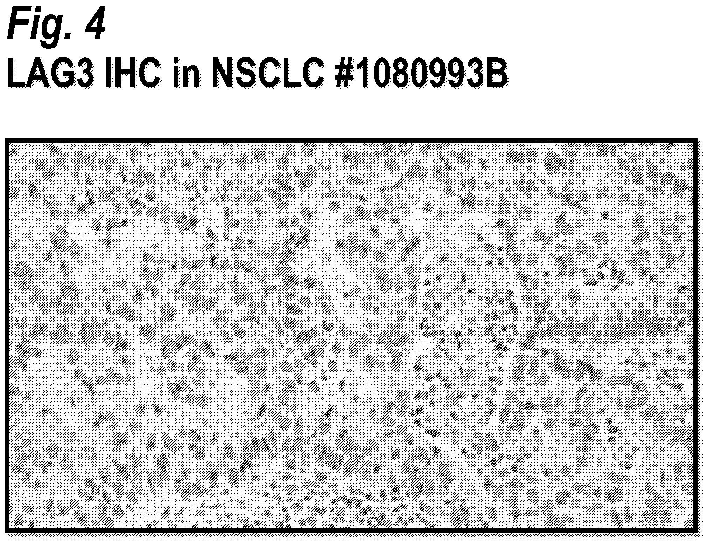

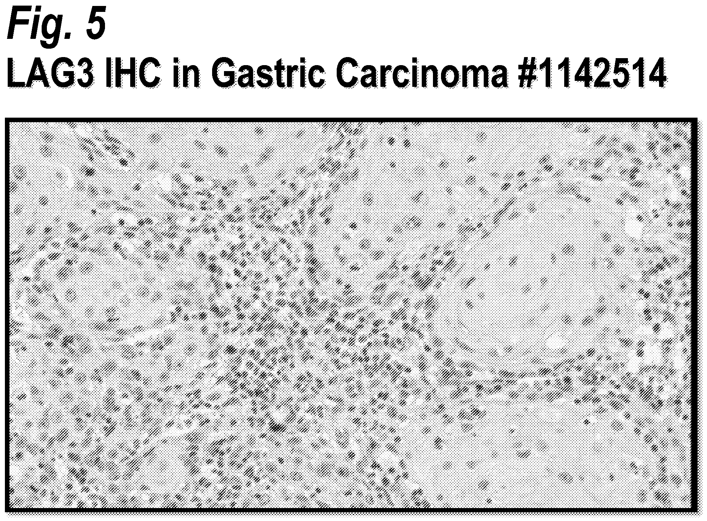

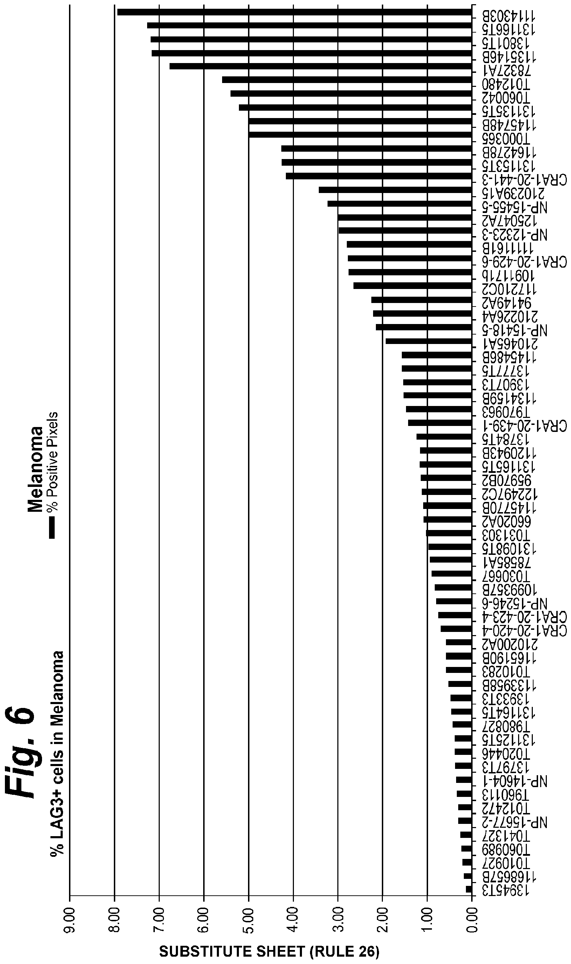

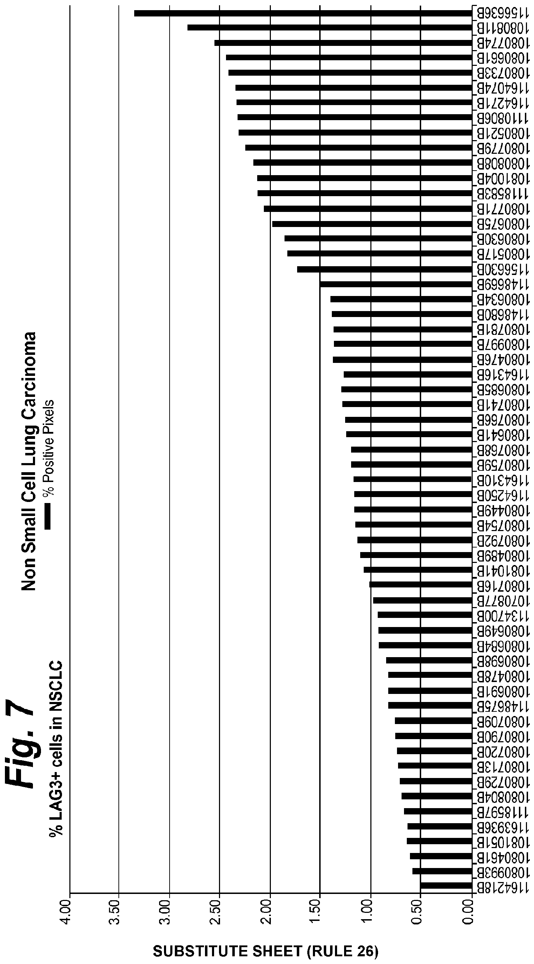

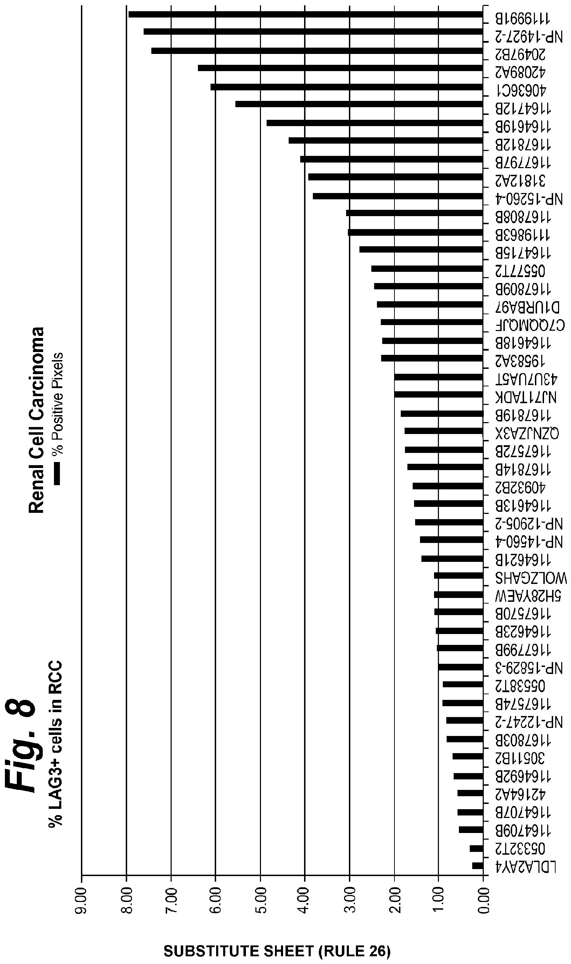

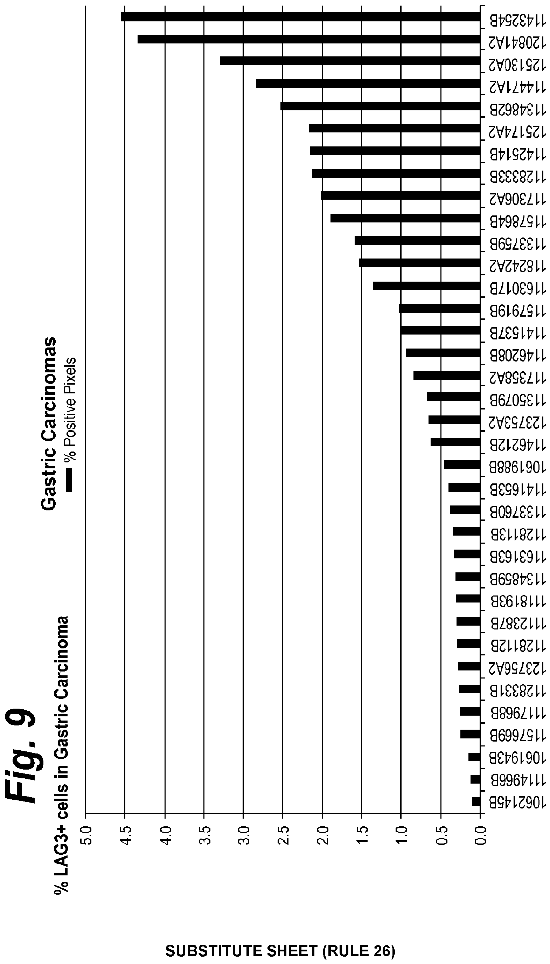

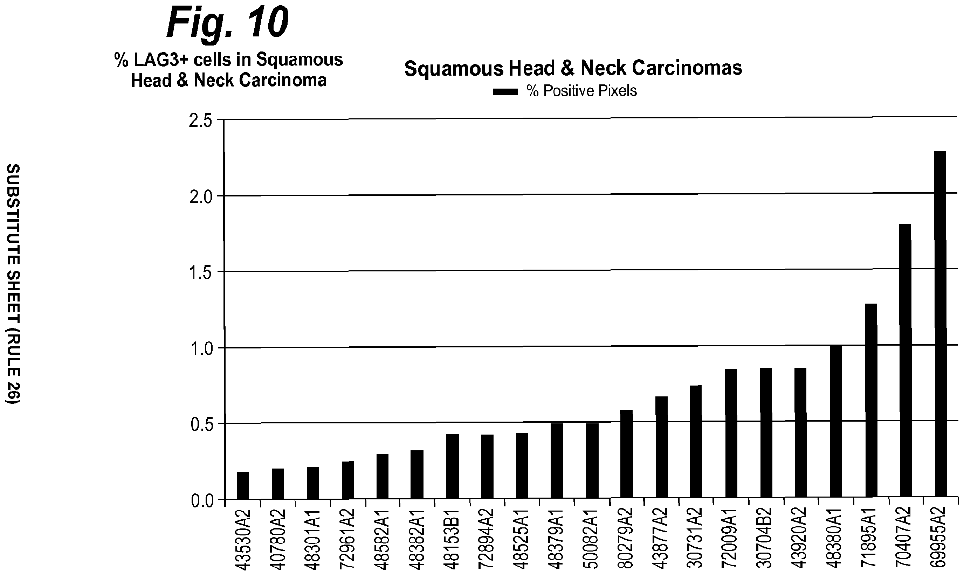

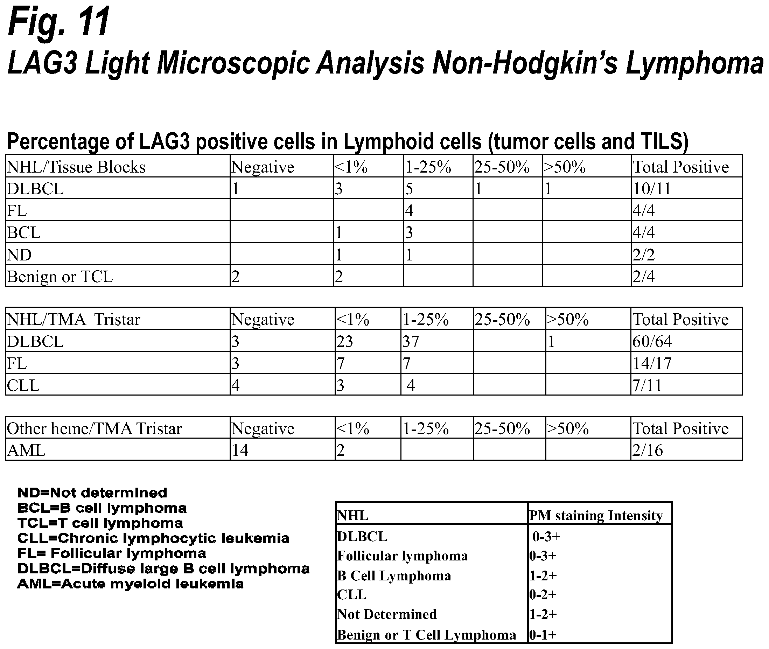

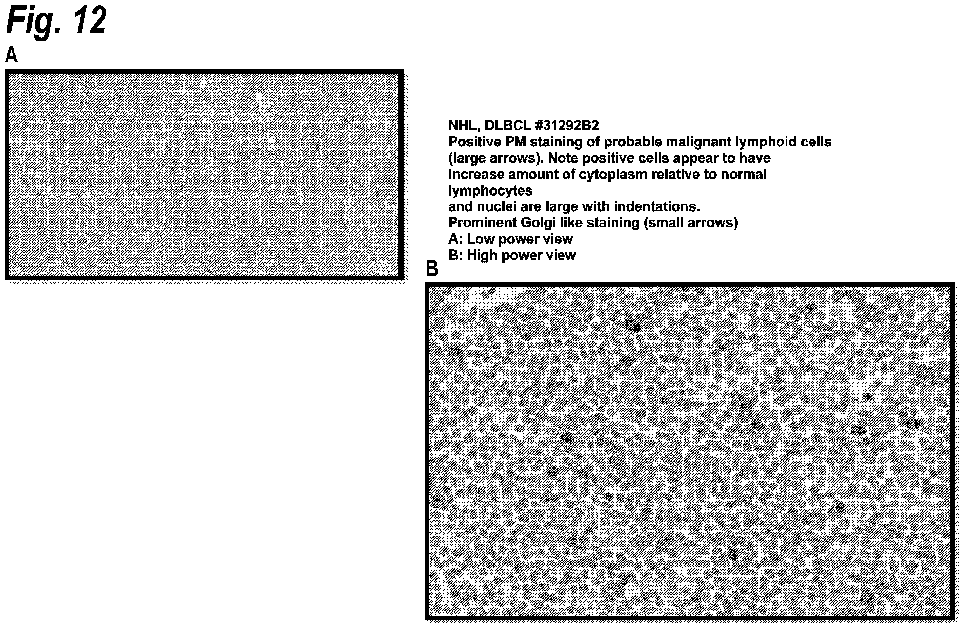

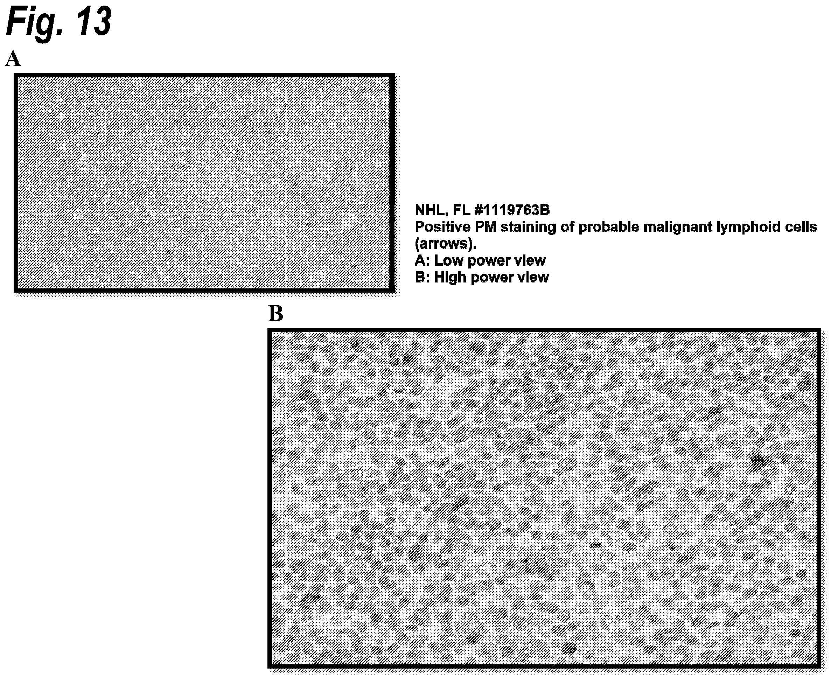

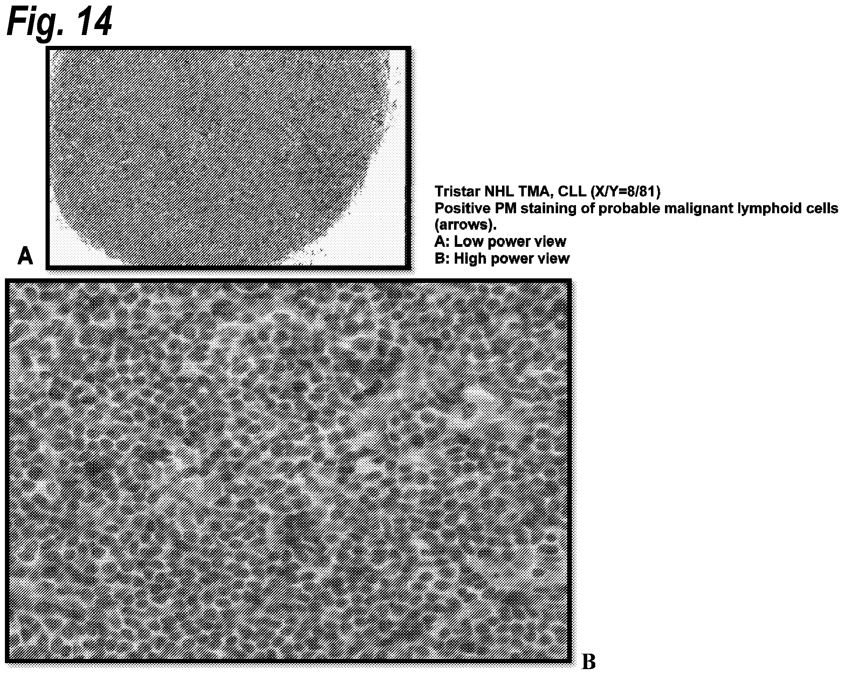

Immunohistochemistry (IHC) will be used to assess the number and composition of immune infiltrates in order to define the immune cell subsets present within FFPE tumor tissue before and after exposure to therapy. These IHC analyses will include, but not necessarily be limited to, the following markers: CD4, CD8, FOXp3, PD-1, LAG-3, and MHC II. Epstein-Barr virus (EBV) status of the tumor may also be performed by assessment of EBV-encoding RNA, LMP-1 expression, or similar assays. For example, IHC analyses for human LAG-3 was conducted on human tonsil and NHL cells according to the following protocol, and these results are shown in Figures 4-14B:

### Equipment and Materials:

Leica autostainer
BioGenex i6000 Autostainer
BioCare Medical Decloaking Chamber™ Plus

### Reagents:

Xylene® (EM Science, Cat # UN1307)
Ethanol (AAPER alcohol and Chemical Co)
AR10 (BioGenex®, Cat# HK057-5K)
Wash Buffer (DAKO®, Cat# S3006)
Peroxidase block (Leica® Cat#RE7101-CE)
Protein block (Leica® Cat#RE7102-CE)
Comment antibody diluent (BioGenex®, Cat# HK156-5K)
DAB (Leica® Cat#RE7190-CE)
Hematoxylin (Dako®, Cat#S3309)

**Antibodies:**

| | | | |
|---|---|---|---|
| LAG-1, Ms Mab | Lifespan® | LS-C18692 | 5 ug/ml |
| Ms IgG1 | R&D® | MAB002 | 5 ug/ml |

| | | | |
|---|---|---|---|
| Novolink Max Polymer Detection System® (Leica®, Cat# RE7260-CE) | | | |

### Procedure:

1. De-paraffin and rehydration was conducted on a Leica autostainer using Xylene (2 times, for 5 minutes each), 100% Ethanol (2 times, for 2 minutes each), 95% Ethanol (2 times, for 2 minutes each), 70% Ethanol (2 times, for 2 minutes each), distilled H₂O (dH₂O) (for 2 minutes);
2. Antigen retrieval was accomplished using Biocare Medical Decloaking Chamber Plus®. The AR10 solution was heated to 105 °C (P1) for 20 minutes, then move to the next step (P2 FAN ON at 95°C; FAN OFF at 90 °C);
3. Slide was cooled at room temperature for 15 minutes, rinsed with water (for approximately 1 minute);
4. IHC reagents were set-up on i6000 autostainer;
5. Slides were set-up on i6000 autostainer, using pap pen to define the tissue area; and
6. IHC was conducted with BioGenex i6000 autostainer using the research mode as follows.

### IHC steps on i6000 (NovoLink Kit):

1. The "Peroxidase block" was applied (NovoLink Kit) for 10 minutes, followed by a rinse with IHC wash buffer (3 times0;
2. The Protein Block (NovoLink Kit) was applied to the slides, and incubated 10 minutes at room temperature, then washed with buffer (3 times);
3. The antibodies were applied to the slides and incubated 1 hour at room temperature and washed with buffer (3 times);
4. The "Post Primary Block" (NovoLink Kit) wasadded to the slides and incubated for 30 minutes and washed with buffer (3 times);
5. The "NovoLink Polymer" (NovoLink Kit) was added to the slides and incubated for 45 minutes;
6. The slides were rinsed with IHC wash buffer (3 times);
7. The DAB chromogen substrates (NovoLink Kit) was added and developed for 5 minutes;
8. The slides were washed by rinsing with dH₂O for 5 times at room temperature;
9. The slides were counterstained with Hematoxylin(Dako) 1:1, for 1 minute at room temperature;
10. Slides were then washed by rinsing with dH₂O (5 times at room temperature).

### De-hydration and coverslipping:

1. Slides were removed from the i6000 autostainer and set-up on the Leica autostainer ;
2. Slides were dehydrated with 70% Ethanol, 95% Ethanol, 2X100% Ethanol (2 minutes each);
3. Slides were washed with Xylene (2 times at 2 minutes each); and
4. Coverslipped with Cytoseal Mounting Medium in Leica CM5030 coverslipper.

### Viral Load in Serum

Epstein-Barr virus (EBV)- and Cytomegalovirus (CMV)- viral load status will be evaluated in serum by (PCR) at different time points. The viral load will then be correlated with the clinical outcomes and the expression of markers such as CD4, CD8, FOXp3, PD-1, LAG-3, and MHC II in tumor tissue.

### Characterization of T-cell repertoire

As described above, DNA sequencing will be performed on pre- and posttreatment tumor tissue to assess the composition of the T-cell repertoire. DNA will be isolated from either the FFPE tumor block or from RNAlater or equivalent preparations.

### Gene expression profiling

Tumor biopsies and bone marrow samples that are collected in RNAlater or equivalent fixative will be examined for mRNA gene expression by Affymetrix gene array technology and/or RT-PCR to detect expression of selected immune-related genes.

### Cytogenetics, Mutations and Tumor Markers

The following analysis need to be performed at baseline if they have not been previously done:
Subjects with Hodgkin Lymphoma: amplification 9p24.1, CD30
Subjects with CLL: del 11q, del 13q, del 17p, IGHV status, CD38 and β2 microglobulin
Subjects with DLBCL: Phenotype ABC, GCB, or unclassifiable and β2 microglobulin.
The results of these clinical markers will be correlated with both, clinical outcomes and the exhausted phenotype in T cells.

### Tumor Sample Collection

### 1. Biopsy (Primary Tumor and/or Lymph Node)

A minimum of 1 FFPE tumor tissue block (preferred) or a minimum of 10 FFPE unstained sections are required for assessment of LAG-3 status, EBER-ISH and other biomarker evaluations. Tumor biopsies in formalin could also be accepted if FFPE is not available.

Biopsy samples should be excisional, incisional, or core needle. Fine needle aspirates or other cytology samples are only allowed after discussion with the Sponsor's Medical Monitor.

It is recommended that samples be fixed in 10% neutral-buffered formalin for 24 to 48 hours.

If slides are submitted, the recommended tissue section thickness is 4 microns and the slides must be positively charged. Slides should be shipped refrigerated at 2-8 °C.

If a fresh biopsy is taken, up to 4 core biopsies are recommended. An assessment of biopsy quality by a pathologist is strongly encouraged at the time of the procedure. The tumor tissue that is obtained from these biopsies will be divided equally into FFPE samples and RNAlater.

The investigator, in consultation with the radiology staff, must determine the degree of risk associated with the procedure and find it acceptable. Biopsies may be done with local anesthesia or conscious sedation. Institutional guidelines for the safe performance of biopsies should be followed. Excisional biopsies may be performed to obtain tumor biopsy samples. Invasive procedures that require general anesthesia should not be performed to obtain a biopsy specimen. However, if a surgical procedure is performed for a clinical indication, excess tumor tissue may be used for research purposes with the consent of the subject.

### 2. Bone Marrow and/or Aspirates

Bone marrow biopsy and/or aspirates taken at baseline, at CR or PR, and at progression will be utilized to assess the phenotypic and functional status of immune cells and tumor cells.

Bone marrow biopsy and/or aspirates will be obtained using institutional standards for these procedures. Detailed instructions for bone marrow biopsy and aspirate collection will be provided in the Laboratory Procedures Manual. In brief, a minimum of 1 FFPE tumor tissue block (preferred) or a minimum of 10 FFPE unstained sections of bone marrow are required and approximately 10 mL of aspirate will be collected in a sodium heparin tube and shipped ambiently. The pathology report should be submitted with the biopsy sample.

### Adverse Events

An adverse event (AE) is defined as any new untoward medical occurrence or worsening of a preexisting medical condition in a clinical investigation subject administered an investigational (medicinal) product and that does not necessarily have a causal relationship with this treatment. An AE is therefore any unfavorable and unintended sign (such as an abnormal laboratory finding), symptom, or disease temporally associated with the use of investigational product, whether or not considered related to the investigational product.

The causal relationship to study drug is determined by a physician and used to assess all adverse events (AE). The casual relationship can be one of the following:
Related: There is a reasonable causal relationship between study drug administration and the AE.
Not related: There is not a reasonable causal relationship between study drug administration and the AE.

### 1. Serious Adverse Events

A serious adverse event (SAE) is any untoward medical occurrence that at any dose:
- results in death
- is life-threatening (defined as an event in which the subject was at risk of death at the time of the event; it does not refer to an event which hypothetically might have caused death if it were more severe)
- requires inpatient hospitalization or causes prolongation of existing hospitalization
- results in persistent or significant disability/incapacity
- is a congenital anomaly/birth defect
- is an important medical event (defined as a medical event(s) that may not be immediately life-threatening or result in death or hospitalization but, based upon appropriate medical and scientific judgment, may jeopardize the subject or may require intervention (*e*.*g*., medical, surgical) to prevent one of the other serious outcomes listed in the definition above). Examples of such events include, but are not limited to, intensive treatment in an emergency room or at home for allergic bronchospasm; blood dyscrasias or convulsions that do not result in hospitalization.) Potential drug induced liver injury (DILI) is also considered an important medical event.

Suspected transmission of an infectious agent (*e*.*g*., pathogenic or nonpathogenic) via the study drug is an SAE. Although pregnancy, overdose, cancer, and potential drug induced liver injury (DILI) are not always serious by regulatory definition, these events must be handled as SAEs. Any component of a study endpoint that is considered related to study therapy (*e*.*g*., death is an endpoint, if death occurred due to anaphylaxis, anaphylaxis must be reported) is reported as SAE.

The following hospitalizations are not considered SAEs:
- a visit to the emergency room or other hospital department < 24 hours, that does not result in admission (unless considered an important medical or life-threatening event)
- elective surgery, planned prior to signing consent
- admissions as per protocol for a planned medical/surgical procedure
- routine health assessment requiring admission for baseline/trending of health status (*e*.*g*., routine colonoscopy)
- medical/surgical admission other than to remedy ill health and planned prior to entry into the study. Appropriate documentation is required in these cases
- admission encountered for another life circumstance that carries no bearing on health status and requires no medical/surgical intervention (*e*.*g*., lack of housing, economic inadequacy, caregiver respite, family circumstances, administrative reason).

Following the subject's written consent to participate in the study, all SAEs, whether related or not related to study drug, are collected, including those thought to be associated with protocol-specified procedures. All SAEs are collected that occur during the screening period and within 135 days of discontinuation of dosing. If applicable, SAEs are collected that relate to any later protocol-specified procedure (*e*.*g*., a follow-up skin biopsy). All SAEs are followed to resolution or stabilization.

### 2. Nonserious Adverse Events

A nonserious adverse event is an AE not classified as serious. The collection of nonserious AE information begins at initiation of study drug and continues for 135 days after discontinuation of dosing. Nonserious AEs are followed to resolution or stabilization, or reported as SAEs if they become serious. Follow-up is also required for nonserious AEs that cause interruption or discontinuation of study drug and for those present at the end of study treatment as appropriate. All identified nonserious AEs are recorded and described on the nonserious AE page of the CRF (paper or electronic).

Completion of supplemental CRFs are requested for AEs and/or laboratory abnormalities that are reported/identified during the course of the study.

### Sample Size Determination

### 1. Part A: Dose Escalation

The sample size at each dose depends on observed toxicity and cannot be precisely determined. Part A will have 3 to 9 subjects in each cohort.

### 2. Part B: Cohort expansion

A sample size of approximately 12 subjects per cohort will allow for better estimation of the toxicity rate and provide greater precision around estimates of preliminary efficacy. If 3 of 12 subjects in a cohort (i.e., < 30%) experience a toxicity there is at least 90% confidence that the true toxicity rate is not greater than 48% (based on Clopper-Pearson exact binomial 1-sided 90% confidence interval). In addition, for a safety signal explored across cohorts, if 14 of 48 subjects (i.e., < 30%) in the expansion portion of the study experience a toxicity, there is at least 90% confidence that the true rate of toxicity does not exceed 40%.

A sample size of approximately 12 subjects per cohort also allows for estimation of the proportion of subjects with objective response (i.e., complete response [CR] + partial response [PR]) within a cohort such that the 2-sided 90% confidence interval for an objective response rate would be 7% to 53% if 3 subjects (25%) had a response, and 12% to 61% if 4 subjects (33%) had a response. In addition, given a true response rate of 15% or higher, there is over an 86% chance of seeing at least one response in a cohort of 12 subjects.

### Populations for Analyses

- **All Enrolled Subjects:** All subjects (including screen failures) who signed an informed consent for the study.
- **All Treated Subjects:** All subjects who receive at least one complete or partial dose of BMS-986016.
- **Response-Evaluable Subjects:** All treated subjects who have an evaluable baseline assessment of disease, and one of the following: (1) at least one evaluable on-treatment disease assessment, (2) clinical progression, or (3) death prior to the first on-treatment disease assessment.

- **Pharmacokinetic Analysis Set:** All treated subjects who and have adequate PK data to define at least one valid PK parameter will be included in summary tables. All available serum concentration data from treated subjects will be listed.
- **Immunogenicity Analysis Set:** All subjects who receive one complete or partial dose of BMS-986016, have a baseline and at least one post-baseline immunogenicity sample available will be included in summary tables. All available data from treated subjects will be listed.
- **Pharmacodynamic Analysis Set:** All treated subjects who have at least one valid measurement for a particular biomarker will be included in summaries and listings for that biomarker.

### Endpoints

### 1. Primary Endpoints

The primary endpoint of this Phase 1 study is safety as measured at the study level by the rate of AEs, SAEs, deaths, and laboratory abnormalities, assessed during treatment and for up to 135 days after the last treatment. All subjects who receive at least one dose of BMS-986016 or nivolumab will be analyzed for safety.

### 2. Secondary Endpoints

### a. Pharmacokinetics

The PK of BMS-986016 will be assessed as a secondary objective using the following endpoints derived from serum concentration versus time data at various timepoints.

**The PK parameters to be assessed include:**

| | |
|---|---|
| Cmax | Maximum observed serum concentration |
| Tmax | Time of maximum observed serum concentration |
| Ctrough | Trough observed serum concentration |
| Ceoinf | Concentration observed at the end of infusion |
| Ctau | Concentration at the end of a dosing interval (e.g., concentration at 336 hours) |
| Css,avg | Average concentration over a dosing interval ([AUC(TAU)/tau] |
| AUC(TAU) | Area under the concentration-time curve in one dosing interval |
| CLT | Total body clearance |
| Vss | Volume of distribution at steady state |
| T-HALFeff AUC | Effective elimination half-life that explains the degree of AUC accumulation observed |
| T-HALFeff Cmax | Effective elimination half-life that explains the degree of Cmax accumulation observed |
| AI_AUC | Accumulation index; ratio of AUC(TAU) at steady state to AUC(TAU) after the first dose |
| AI_Cmax | Cmax accumulation index; ratio of Cmax at steady state to Cmax after the first dose |
| AI_Ctau | Ctau accumulation index; ratio of Ctau at steady state to Ctau after the first dose |
| AI_Ceoinf | Ceoinf accumulation index; ratio of Ceoinf at steady state to Ceoinf after the first dose |
| DF | Degree of fluctuation or fluctuation index ([Cmax - Ctau]/Css,avg) |

Individual subject PK parameter values are derived by noncompartmental methods by a validated PK analysis program. Actual times are used for the analyses.

### b. Efficacy

The following secondary endpoints will be assessed to investigate the preliminary antitumor activity of BMS-986016:
- Objective response rate (ORR), i.e., the proportion of subjects with a best overall response of CR or PR
- Duration of response (DOR), based on current criteria relevant to each disease type
- Landmark progression-free survival rate (PFSR) at 4, 6, 8, and 10 months on treatment and 30 days after the last dose will be evaluated as an exploratory endpoint.

### 3. Exploratory Endpoints / Biomarkers

Biomarkers endpoints from peripheral blood may include measures such as levels of soluble factors, as well as subsets of T cells characterized by immunophenotyping, at each scheduled timepoint. Biomarker endpoints from tumor biopsies may include, but will not be limited to, measures such as functional status and arrangement of lymphocytes and lymphocyte activation gene 3 (LAG-3), major histocompatibility complex (MHC) class II, programmed cell death 1 (PD-1), and programmed cell death ligand 1 (PD-L1) expression. Measures of receptor occupancy as characterized in peripheral blood, bone marrow, and lymph node (if available) may also be provided.

### Analysis

Unless otherwise specified, safety data from Parts A and B will be summarized both: 1) overall by dose level and across all dose levels and also, 2) by dose level and across all dose levels within each tumor type. Efficacy data will be summarized by dose level within each tumor type.

### 1. Demographics and Baseline Characteristics

Frequency distributions of gender and race will be tabulated. Summary statistics for age, body weight, height, and body mass index (BMI) will be tabulated.

### 2. Efficacy Analyses

In subjects with HL and NHL, the Revised Response Criteria for Malignant Lymphoma (Appendix 1) will be used to evaluate efficacy. For subjects with CLL, the Guidelines for the Diagnosis and Treatment of Chronic Lymphocytic Leukemia will be used.

Individual BOR, ORR, DOR, and PFSR at selected timepoints will be determined. BOR outcomes will be summarized using frequency tables. The ORR, landmark PFSR (at 4, 6, 8, and 10 months on treatment and 30 days after the last dose) and the corresponding confidence intervals will be calculated for Part B and may be calculated for Part A as supported by the data. The DOR and PFSR will be estimated by tumor type using Kaplan-Meier methodology. Additional exploratory presentations of efficacy may include subjects in both dose escalation and cohort expansion grouped by tumor type, treatment, prior exposure to immunotherapy, or baseline tumor markers. Plots of individual change in disease burden over time will also be produced. Individual changes in tumor markers over time may be presented graphically by dose level within select disease types. Depending on the purpose of the analysis, efficacy may be reported for either all treated subjects or response-evaluable subjects.

### 3. Safety Analyses

All subjects who receive study drug therapy will be included in the analysis of safety endpoints. All recorded AEs will be listed and tabulated by system organ class, preferred term, relationship to study drug, and treatment. Coding will be done according to the most current version of MedDRA. Vital signs and select clinical laboratory test results will be listed and summarized by treatment. Any significant physical examination findings and results of clinical laboratory tests will be listed. Any ECG abnormalities identified by the Investigator will be listed.

### 4. Pharmacokinetic Analyses

PK parameters for BMS-986016 will be calculated using noncompartmental analyses. Summary statistics will be tabulated for the PK parameters of BMS-986016 by treatment and study day/week. To describe the dependency on dose of BMS-986016, scatter plots of Cmax and AUC (TAU) versus dose may be provided for each day measured. Dose proportionality of BMS- 986016 may also be assessed based on a power model.

### 5. Exploratory Biomarker Analyses

The pharmacodynamic effect on TILs, MILs, and other key tumor markers in subjects who undergo biopsy will be summarized using summary statistics and plots. In addition, the correlation of TIL or MIL changes and tumor marker expression with measures of peripheral blood markers may be explored graphically, and using appropriate modeling approaches based on data availability. Associations of biomarker measures from peripheral blood or tumor biopsy with clinical outcomes may also be explored graphically and further assessed as needed by methods such as, but not limited to, logistic regression and characterized by appropriate statistics.

### 6. Immunogenicity Analyses

A listing will be provided for all available immunogenicity data. The number and percent of subjects who meet specified endpoint definitions will be summarized. To examine the potential relationship between immunogenicity and safety, a table summarizing the frequency and type of AEs of special interest may be explored by immunogenicity status. In addition, potential relationships between immunogenicity and efficacy, pharmacodynamic markers, and/or PK may also be explored.

**SEQUENCE SUMMARY**

| **SEQ ID NO:** | **SEQUENCE** |
|---|---|
| 1 | |
| | Heavy Chain Amino Acid SequenceAnti-LAG-3 mAb (BMS-986016)(variable region underlined; constant region bold) |
| | |
| 2 | Light Chain Amino Acid Sequence |
| | Anti-LAG-3 mAb (BMS-986016) (variable region underlined; constant region bold) |
| | |
| 3 | Heavy Chain Variable Region (VH) Amino Acid Sequence |
| | Anti-LAG-3 mAb (BMS-986016) |
| | |
| 4 | Heavy Chain Variable Region (VH) Nucleotide Sequence |
| | Anti-LAG-3 mAb (BMS-986016) |
| | |
| 5 | Light Chain Variable Region (VL) Amino Acid Sequence |
| | Anti-LAG-3 mAb (BMS-986016) |
| | |
| | |
| 6 | Light Chain Variable Region (VL) Nucleotide Sequence |
| | Anti-LAG-3 mAb (BMS-986016) |
| | |
| 7 | Heavy Chain CDR1 Amino Acid Sequence |
| | Anti-LAG-3 mAb (BMS-986016) |
| | DYYWN |
| 8 | Heavy Chain CDR2 Amino Acid Sequence |
| | Anti-LAG-3 mAb (BMS-986016) |
| | EINHRGSTNSNPSLKS |
| 9 | Heavy Chain CDR3 Amino Acid Sequence |
| | Anti-LAG-3 mAb (BMS-986016) |
| | GYSDYEYNWFDP |
| 10 | Light Chain CDR1 Amino Acid Sequence |
| | Anti-LAG-3 mAb (BMS-986016) |
| | RASQSISSYLA |
| 11 | Light Chain CDR2 Amino Acid Sequence |
| | Anti-LAG-3 mAb (BMS-986016) |
| | DASNRAT |
| 12 | Light Chain CDR3 Amino Acid Sequence |
| | Anti-LAG-3 mAb (BMS-986016) |
| | QQRSNWPLT |
| 13 | Human LAG-3 Amino Acid Sequence |
| | |
| 14 | LAG-3 Epitope |
| | PGHPLAPG |
| 15 | LAG-3 Epitope |
| | HPAAPSSW |
| 16 | LAG-3 Epitope |
| | PAAPSSWG |
| 17 | Heavy Chain Nucleotide Sequence |
| | Anti-LAG-3 mAb (BMS-986016) |
| | |
| | |
| 18 | Light Chain Nucleotide Sequence |
| | Anti-LAG-3 mAb (BMS-986016) |
| | |

## Claims

1. An anti-LAG-3 antibody for use in a method of treating a hematological malignancy in a human patient, wherein the anti-LAG-3 antibody comprises
(a) a heavy chain variable region CDR1 having the sequence set forth in SEQ ID NO:7;
(b) a heavy chain variable region CDR2 having the sequence set forth in SEQ ID NO:8;
(c) a heavy chain variable region CDR3 having the sequence set forth in SEQ ID NO:9;
(d) a light chain variable region CDR1 having the sequence set forth in SEQ ID NO:10;
(e) a light chain variable region CDR2 having the sequence set forth in SEQ ID NO:11; and
(f) a light chain variable region CDR3 having the sequence set forth in SEQ ID NO:12, and
wherein the method comprises at least one administration cycle of the anti-LAG-3 antibody, wherein the cycle is a period of eight weeks, wherein for each of the at least one cycles, the anti-LAG-3 antibody is administered as follows:
(a) four doses of 20 mg anti-LAG-3 antibody;
(b) four doses of 80 mg anti-LAG-3 antibody;
(c) four doses of 240 mg anti-LAG-3 antibody; or
(d) four doses of 800 mg anti-LAG-3 antibody.

2. The anti-LAG-3 antibody for use according to claim 1, wherein the anti-LAG-3 antibody is formulated for intravenous administration.

3. The anti-LAG-3 antibody for use according to claim 1 or 2, wherein the treatment consists of up to 12 cycles.

4. The anti-LAG-3 antibody for use according to any one of claims 1-3, wherein the method comprises administration of the anti-LAG-3 antibody on Days 1, 15, 29, and 43 of each cycle.

5. The anti-LAG-3 antibody for use according to any one of claims 1-4, wherein the treatment produces at least one therapeutic effect chosen from a reduction in the number of malignant cells, inhibition of malignant cell infiltration, inhibition of malignant cell metastasis, prevention occurrence of malignant cells, and reduction of one or more of the symptoms associated with the malignancy.

6. The anti-LAG-3 antibody for use according to any one of claims 1-5, wherein the malignancy is chosen from a leukemia, lymphoma, and myeloma.

7. The anti-LAG-3 antibody for use according to any one of claims 1-6, wherein the malignancy is chosen from a relapsed or refractory chronic lymphocytic leukemia and lymphoma.

8. The anti-LAG-3 antibody for use according to claim 7, wherein the malignancy is chosen from chronic lymphocytic leukemia (CLL), Hodgkin lymphoma (HL), and non-Hodgkin lymphoma (NHL).

9. The anti-LAG-3 antibody for use according to any one of claims 1-8, wherein the anti-LAG-3 antibody comprises heavy and light chain variable regions having the sequences set forth in SEQ ID NOs:3 and 5, respectively.

10. The anti-LAG-3 antibody for use according to any one of claims 1-9, wherein the anti-LAG-3 antibody comprises heavy and light chains having the sequences set forth in SEQ ID NOs:1 and 2, respectively.

11. The anti-LAG-3 antibody for use according to any one of claims 1-10, wherein the anti-LAG-3 antibody has an IgG4 isotype that includes a S228P mutation.

12. The anti-LAG-3 antibody for use according to any one of claims 1-11, wherein the method further comprises using immunohistochemistry to assess the number and composition of immune filtrates in order to define the immune cell subsets present within formalin-fixed paraffin-embedded tumor tissue samples collected from the human patient before and after exposure to the anti-LAG-3 antibody.

13. The anti-LAG-3 antibody for use according to any one of claims 1-11, wherein the method further comprises analyzing the expression levels of LAG-3, PD-1, PD-L1, PD-L2, ICOS and Ki67 in a sample of peripheral blood mononuclear cells or bone marrow aspirate of the human patient collected prior to therapy with the anti-LAG-3 antibody.

## Patentansprüche

1. Anti-LAG-3-Antikörper zur Verwendung in einem Verfahren zum Behandeln einer hämatologischen Malignität bei einem menschlichen Patienten, wobei der Anti-LAG-3-Antikörper umfasst:
(a) eine variable Region der schweren Kette CDR1 mit der Sequenz, die in SEQ ID NO: 7 angegeben ist;
(b) eine variable Region der schweren Kette CDR2 mit der Sequenz, die in SEQ ID NO: 8 angegeben ist;
(c) eine variable Region der schweren Kette CDR3 mit der Sequenz, die in SEQ ID NO: 9 angegeben ist;
(d) eine variable Region der leichten Kette CDR1 mit der Sequenz, die in SEQ ID NO: 10 angegeben ist;
(e) eine variable Region der leichten Kette CDR2 mit der Sequenz, die in SEQ ID NO: 11 angegeben ist; und
(f) eine variable Region der leichten Kette CDR3 mit der Sequenz, die in SEQ ID NO: 12 angegeben ist, und
wobei das Verfahren mindestens einen Verabreichungszyklus des Anti-LAG-3-Antikörpers umfasst, wobei der Zyklus einen Zeitraum von acht Wochen umfasst, wobei für jeden des mindestens einen Zyklus der Anti-LAG-3-Antikörper wie folgt verabreicht wird:
(a) vier Dosen von 20 mg Anti-LAG-3-Antikörper;
(b) vier Dosen von 80 mg Anti-LAG-3-Antikörper;
(c) vier Dosen von 240 mg Anti-LAG-3-Antikörper; oder
(d) vier Dosen von 800 mg Anti-LAG-3-Antikörper.

2. Anti-LAG-3-Antikörper zur Verwendung nach Anspruch 1, wobei der Anti-LAG-3-Antikörper zur intravenösen Verabreichung formuliert ist.

3. Anti-LAG-3-Antikörper zur Verwendung nach Anspruch 1 oder 2, wobei die Behandlung aus bis zu 12 Zyklen besteht.

4. Anti-LAG-3-Antikörper zur Verwendung nach einem der Ansprüche 1 bis 3, wobei das Verfahren die Verabreichung des Anti-LAG-3-Antikörpers an den Tagen 1, 15, 29 und 43 von jedem Zyklus umfasst.

5. Anti-LAG-3-Antikörper zur Verwendung nach einem der Ansprüche 1 bis 4, wobei die Behandlung mindestens eine therapeutische Wirkung erzeugt, die ausgewählt ist aus einer Verringerung der Anzahl von malignen Zellen, Hemmung der Infiltration von malignen Zellen, Hemmung von malignen Zellmetastasen, Verhinderung des Auftretens von malignen Zellen und Verringerung eines oder mehrerer der Symptome, die mit der Malignität im Zusammenhang stehen.

6. Anti-LAG-3-Antikörper zur Verwendung nach einem der Ansprüche 1 bis 5, wobei die Malignität aus Leukämie, Lymphom und Myelom gewählt ist.

7. Anti-LAG-3-Antikörper zur Verwendung nach einem der Ansprüche 1 bis 6, wobei die Malignität aus einer rezidivierten oder refraktären chronischen lymphatischen Leukämie und einem Lymphom gewählt ist.

8. Anti-LAG-3-Antikörper zur Verwendung nach Anspruch 7, wobei die Malignität ausgewählt ist aus chronischer lymphatischer Leukämie (CLL), Hodgkin-Lymphom (HL) und Non-Hodgkin-Lymphom (NHL).

9. Anti-LAG-3-Antikörper zur Verwendung nach einem der Ansprüche 1 bis 8, wobei der Anti-LAG-3-Antikörper variable Regionen der schweren und leichten Kette umfassen, welche die in SEQ ID NO: 3 bzw. 5 angegebenen Sequenzen aufweisen.

10. Anti-LAG-3-Antikörper zur Verwendung nach einem der Ansprüche 1 bis 9, wobei der Anti-LAG-3-Antikörper schwere und leichte Ketten umfasst, welche die in SEQ ID NO: 1 bzw. 2 angegebenen Sequenzen aufweisen.

11. Anti-LAG-3-Antikörper zur Verwendung nach einem der Ansprüche 1 bis 10, wobei der Anti-LAG-3-Antikörper einen IGg4-Isotyp aufweist, der eine S228P-Mutation einschließt.

12. Anti-LAG-3-Antikörper zur Verwendung nach einem der Ansprüche 1 bis 11, wobei das Verfahren ferner die Anwendung von Immunhistochemie umfasst, um die Anzahl und Zusammensetzung von Immunfiltraten zu bestimmen, um die Untergruppen von Immunzellen zu festzulegen, die in mit Formalin fixierten, in Paraffin eingebetteten Tumorgewebeproben vorhanden sind, die dem menschlichen Patienten vor und nach der Exponierung an dem Anti-LAG-3-Antikörper entnommen wurden.

13. Anti-LAG-3-Antikörper zur Verwendung nach einem der Ansprüche 1 bis 11, wobei das Verfahren ferner Analysieren der Expressionsniveaus von LAG-3, PD-1, PD-L1, PD-L2, ICOS und Ki67 in einer Probe von peripheren mononuklearen Blutzellen oder Knochenmarkaspirat des menschlichen Patienten umfasst, die vor der Therapie mit dem Anti-LAG-3-Antikörper entnommen wurden.

## Revendications

1. Anticorps anti-LAG-3 pour une utilisation dans une méthode de traitement d'une malignité hématologique chez un patient humain, où l'anticorps anti-LAG-3 comprend:
(a) une CDR1 de région variable de chaîne lourde possédant la séquence présentée dans la SEQ ID NO: 7;
(b) une CDR2 de région variable de chaîne lourde possédant la séquence présentée dans la SEQ ID NO: 8;
(c) une CDR3 de région variable de chaîne lourde possédant la séquence présentée dans la SEQ ID NO: 9;
(d) une CDR1 de région variable de chaîne légère possédant la séquence présentée dans la SEQ ID NO: 10;
(e) une CDR2 de région variable de chaîne légère possédant la séquence présentée dans la SEQ ID NO: 11; et
(f) une CDR3 de région variable de chaîne légère possédant la séquence présentée dans la SEQ ID NO: 12, et
où la méthode comprend au moins un cycle d'administration de l'anticorps anti-LAG-3, où le cycle est une période de huit semaines, où pour chacun du au moins un cycle, l'anticorps anti-LAG-3 est administré comme suit:
(a) quatre doses de 20 mg d'anticorps anti-LAG-3;
(b) quatre doses de 80 mg d'anticorps anti-LAG-3;
(c) quatre doses de 240 mg d'anticorps anti-LAG-3; ou
(d) quatre doses de 800 mg d'anticorps anti-LAG-3.

2. Anticorps anti-LAG-3 pour une utilisation selon la revendication 1, où l'anticorps anti-LAG-3 est formulé pour une administration intraveineuse.

3. Anticorps anti-LAG-3 pour une utilisation selon la revendication 1 ou 2, où le traitement est constitué de jusqu'à 12 cycles.

4. Anticorps anti-LAG-3 pour une utilisation selon l'une quelconque des revendications 1-3, où la méthode comprend l'administration de l'anticorps anti-LAG-3 les jours 1, 15, 29 et 43 de chaque cycle.

5. Anticorps anti-LAG-3 pour une utilisation selon l'une quelconque des revendications 1-4, où le traitement produit au moins un effet thérapeutique choisi parmi une réduction du nombre de cellules malignes, une inhibition de l'infiltration de cellules malignes, une inhibition de la métastase de cellules malignes, une prévention de l'occurrence de cellules malignes et une réduction d'un ou de plusieurs des symptômes associés à la malignité.

6. Anticorps anti-LAG-3 pour une utilisation selon l'une quelconque des revendications 1-5, où la malignité est choisie parmi une leucémie, un lymphome et un myélome.

7. Anticorps anti-LAG-3 pour une utilisation selon l'une quelconque des revendications 1-6, où la malignité est choisie parmi une leucémie lymphoïde chronique en rechute ou réfractaire et un lymphome.

8. Anticorps anti-LAG-3 pour une utilisation selon la revendication 7, où la malignité est choisie parmi une leucémie lymphoïde chronique (CLL), un lymphome hodgkinien (HL) et un lymphome non hodgkinien (NHL).

9. Anticorps anti-LAG-3 pour une utilisation selon l'une quelconque des revendications 1-8, où l'anticorps anti-LAG-3 comprend des régions variables de chaînes lourde et légère possédant les séquences présentées dans les SEQ ID NOs: 3 et 5, respectivement.

10. Anticorps anti-LAG-3 pour une utilisation selon l'une quelconque des revendications 1-9, où l'anticorps anti-LAG-3 comprend des chaînes lourde et légère possédant les séquences présentées dans les SEQ ID NOs: 1 et 2, respectivement.

11. Anticorps anti-LAG-3 pour une utilisation selon l'une quelconque des revendications 1-10, où l'anticorps anti-LAG-3 a un isotype IgG4 qui inclut une mutation S228P.

12. Anticorps anti-LAG-3 pour une utilisation selon l'une quelconque des revendications 1-11, où la méthode comprend en outre l'utilisation d'une immunohistochimie pour évaluer le nombre et la composition de filtrats immunitaires dans le but de définir les sous-groupes de cellules immunitaires présentes dans des échantillons de tissus tumoraux noyés dans la paraffine fixés à la formaline récupérés à partir du patient humain avant et après une exposition à l'anticorps anti-LAG-3.

13. Anticorps anti-LAG-3 pour une utilisation selon l'une quelconque des revendications 1-11, où la méthode comprend en outre l'analyse des niveaux d'expression de LAG-3, PD-1, PD-L1, PD-L2, ICOS et Ki67 dans un échantillon de cellules mononucléaires de sang périphérique ou un prélèvement par aspiration de moelle osseuse du patient humain récupérés avant une thérapie avec l'anticorps anti-LAG-3.


### SEQUENCE LISTING

<110> BRISTOL-MYERS SQUIBB COMPANY
<120> ANTI-LAG-3 ANTIBODIES TO TREAT HEMATOLOGICAL MALIGNANCIES
<130> M/57184-EP-DIV1
<140> EP 15705137.6
   <141> 2015-01-26
<150> US 61/932,589
   <151> 2014-01-28
<160> 18
<170> PatentIn version 3.5
<210> 1
   <211> 447
   <212> PRT
   <213> Artificial Sequence
<220>
   <223> Synthetic: Heavy Chain Amino Acid Sequence, Anti-LAG-3 mAb (BMS-986016)
<400> 1
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Asp Tyr
               20                  25                  30
   Tyr Trp Asn Trp Ile Arg Gln Pro Pro Gly Lys Gly Leu Glu Trp Ile
           35                  40                  45
   Gly Glu Ile Asn His Arg Gly Ser Thr Asn Ser Asn Pro Ser Leu Lys
       50                  55                  60
   Ser Arg Val Thr Leu Ser Leu Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Phe Gly Tyr Ser Asp Tyr Glu Tyr Asn Trp Phe Asp Pro Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Cys Ser Arg Ser Thr Ser Glu Ser Thr Ala Ala
       130                 135                 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Lys Thr Tyr Thr Cys Asn Val Asp His Lys
   195 200 205
   Pro Ser Asn Thr Lys Val Asp Lys Arg Val Glu Ser Lys Tyr Gly Pro
   210 215 220
   Pro Cys Pro Pro Cys Pro Ala Pro Glu Phe Leu Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser Gln Glu Asp Pro Glu
   260 265 270
   Val Gln Phe Asn Trp Tyr Val Asp Gly Val Glu Val His Asn Ala Lys
   275 280 285
   Thr Lys Pro Arg Glu Glu Gln Phe Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Gly Leu Pro Ser Ser Ile Glu Lys Thr Ile
   325 330 335
   Ser Lys Ala Lys Gly Gln Pro Arg Glu Pro Gln Val Tyr Thr Leu Pro
   340 345 350
   Pro Ser Gln Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Arg Leu Thr Val Asp Lys Ser Arg
   405 410 415
   Trp Gln Glu Gly Asn Val Phe Ser Cys Ser Val Met His Glu Ala Leu
   420 425 430
   His Asn His Tyr Thr Gln Lys Ser Leu Ser Leu Ser Leu Gly Lys
   435 440 445
<210> 2
   <211> 214
   <212> PRT
   <213> Artificial Sequence
<220>
   <223> Synthetic: Light Chain Amino Acid Sequence, Anti-LAG-3 mAb (BMS-986016)
<400> 2
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Ile Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Leu
                   85                  90                  95
   Thr Phe Gly Gln Gly Thr Asn Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 120
   <212> PRT
   <213> Artificial Sequence
<220>
   <223> Synthetic: Heavy Chain Variable Region (VH) Amino Acid Sequence, Anti-LAG-3 mAb (BMS-986016)
<400> 3
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Asp Tyr
               20                  25                  30
   Tyr Trp Asn Trp Ile Arg Gln Pro Pro Gly Lys Gly Leu Glu Trp Ile
           35                  40                  45
   Gly Glu Ile Asn His Arg Gly Ser Thr Asn Ser Asn Pro Ser Leu Lys
       50                  55                  60
   Ser Arg Val Thr Leu Ser Leu Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Phe Gly Tyr Ser Asp Tyr Glu Tyr Asn Trp Phe Asp Pro Trp Gly Gln
               100                 105                 110
   Gly Thr Leu Val Thr Val Ser Ser
           115                 120
<210> 4
   <211> 360
   <212> DNA
   <213> Artificial Sequence
<220>
   <223> Synthetic: Heavy Chain Variable Region (VH) Nucleotide Sequence, Anti-LAG-3 mAb (BMS-986016)
<400> 4
   caggtgcagc tacagcagtg gggcgcagga ctgttgaagc cttcggagac cctgtccctc      60
   acctgcgctg tctatggtgg gtccttcagt gattactact ggaactggat ccgccagccc     120
   ccagggaagg ggctggagtg gattggggaa atcaatcatc gtggaagcac caactccaac     180
   ccgtccctca agagtcgagt caccctatca ctagacacgt ccaagaacca gttctccctg     240
   aagctgaggt ctgtgaccgc cgcggacacg gctgtgtatt actgtgcgtt tggatatagt     300
   gactacgagt acaactggtt cgacccctgg ggccagggaa ccctggtcac cgtctcctca     360
<210> 5
   <211> 107
   <212> PRT
   <213> Artificial Sequence
<220>
   <223> Synthetic: Light Chain Variable Region (VL) Amino Acid Sequence, Anti-LAG-3 mAb (BMS-986016)
<400> 5
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Ile Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Leu
                   85                  90                  95
   Thr Phe Gly Gln Gly Thr Asn Leu Glu Ile Lys
               100                 105
<210> 6
   <211> 321
   <212> DNA
   <213> Artificial Sequence
<220>
   <223> Synthetic: Light Chain Variable Region (VL) Nucleotide Sequence, Anti-LAG-3 mAb (BMS-986016)
<400> 6
   gaaattgtgt tgacacagtc tccagccacc ctgtctttgt ctccagggga aagagccacc       60
   ctctcctgca gggccagtca gagtattagc agctacttag cctggtacca acagaaacct      120
   ggccaggctc ccaggctcct catctatgat gcatccaaca gggccactgg catcccagcc      180
   aggttcagtg gcagtgggtc tgggacagac ttcactctca ccatcagcag cctagagcct      240
   gaagattttg cagtttatta ctgtcagcag cgtagcaact ggcctctcac ttttggccag      300
   gggaccaacc tggagatcaa a                                                321
<210> 7
   <211> 5
   <212> PRT
   <213> Artificial Sequence
<220>
   <223> Synthetic: Heavy Chain CDR1 Amino Acid Sequence , Anti-LAG-3 mAb (BMS-986016)
<400> 7
   Asp Tyr Tyr Trp Asn
   1               5
<210> 8
   <211> 16
   <212> PRT
   <213> Artificial Sequence
<220>
   <223> Synthetic: Heavy Chain CDR2 Amino Acid Sequence, Anti-LAG-3 mAb (BMS-986016)
<400> 8
   Glu Ile Asn His Arg Gly Ser Thr Asn Ser Asn Pro Ser Leu Lys Ser
   1               5                   10                  15
<210> 9
   <211> 12
   <212> PRT
   <213> Artificial Sequence
<220>
   <223> Synthetic: Heavy Chain CDR3 Amino Acid Sequence, Anti-LAG-3 mAb (BMS-986016)
<400> 9
   Gly Tyr Ser Asp Tyr Glu Tyr Asn Trp Phe Asp Pro
       1               5                   10
<210> 10
   <211> 11
   <212> PRT
   <213> Artificial Sequence
<220>
   <223> Synthetic: Light Chain CDR1 Amino Acid Sequence, Anti-LAG-3 mAb (BMS-986016)
<400> 10
   Arg Ala Ser Gln Ser Ile Ser Ser Tyr Leu Ala
   1               5                   10
<210> 11
   <211> 7
   <212> PRT
   <213> Artificial Sequence
<220>
   <223> Synthetic: Light Chain CDR2 Amino Acid Sequence, Anti-LAG-3 mAb (BMS-986016)
<400> 11
   Asp Ala Ser Asn Arg Ala Thr
   1               5
<210> 12
   <211> 9
   <212> PRT
   <213> Artificial Sequence
<220>
   <223> Synthetic: Light Chain CDR3 Amino Acid Sequence, Anti-LAG-3 mAb (BMS-986016)
<400> 12
   Gln Gln Arg Ser Asn Trp Pro Leu Thr
   1               5
<210> 13
   <211> 525
   <212> PRT
   <213> Homo sapiens
<220>
   <221> misc_feature
   <223> Human LAG-3 Amino Acid Sequence
<400> 13
   Met Trp Glu Ala Gln Phe Leu Gly Leu Leu Phe Leu Gln Pro Leu Trp
   1               5                   10                  15
   Val Ala Pro Val Lys Pro Leu Gln Pro Gly Ala Glu Val Pro Val Val
               20                  25                  30
   Trp Ala Gln Glu Gly Ala Pro Ala Gln Leu Pro Cys Ser Pro Thr Ile
           35                  40                  45
   Pro Leu Gln Asp Leu Ser Leu Leu Arg Arg Ala Gly Val Thr Trp Gln
       50                  55                  60
   His Gln Pro Asp Ser Gly Pro Pro Ala Ala Ala Pro Gly His Pro Leu
   65                  70                  75                  80
   Ala Pro Gly Pro His Pro Ala Ala Pro Ser Ser Trp Gly Pro Arg Pro
                   85                  90                  95
   Arg Arg Tyr Thr Val Leu Ser Val Gly Pro Gly Gly Leu Arg Ser Gly
               100                 105                 110
   Arg Leu Pro Leu Gln Pro Arg Val Gln Leu Asp Glu Arg Gly Arg Gln
           115                 120                 125
   Arg Gly Asp Phe Ser Leu Trp Leu Arg Pro Ala Arg Arg Ala Asp Ala
       130                 135                 140
   Gly Glu Tyr Arg Ala Ala Val His Leu Arg Asp Arg Ala Leu Ser Cys
   145                 150                 155                 160
   Arg Leu Arg Leu Arg Leu Gly Gln Ala Ser Met Thr Ala Ser Pro Pro
                   165                 170                 175
   Gly Ser Leu Arg Ala Ser Asp Trp Val Ile Leu Asn Cys Ser Phe Ser
               180                 185                 190
   Arg Pro Asp Arg Pro Ala Ser Val His Trp Phe Arg Asn Arg Gly Gln
           195                 200                 205
   Gly Arg Val Pro Val Arg Glu Ser Pro His His His Leu Ala Glu Ser
       210                 215                 220
   Phe Leu Phe Leu Pro Gln Val Ser Pro Met Asp Ser Gly Pro Trp Gly
   225                 230                 235                 240
   Cys Ile Leu Thr Tyr Arg Asp Gly Phe Asn Val Ser Ile Met Tyr Asn
                   245                 250                 255
   Leu Thr Val Leu Gly Leu Glu Pro Pro Thr Pro Leu Thr Val Tyr Ala
               260                 265                 270
   Gly Ala Gly Ser Arg Val Gly Leu Pro Cys Arg Leu Pro Ala Gly Val
   275 280 285
   Gly Thr Arg Ser Phe Leu Thr Ala Lys Trp Thr Pro Pro Gly Gly Gly
   290 295 300
   Pro Asp Leu Leu Val Thr Gly Asp Asn Gly Asp Phe Thr Leu Arg Leu
   305 310 315 320
   Glu Asp Val Ser Gln Ala Gln Ala Gly Thr Tyr Thr Cys His Ile His
   325 330 335
   Leu Gln Glu Gln Gln Leu Asn Ala Thr Val Thr Leu Ala Ile Ile Thr
   340 345 350
   Val Thr Pro Lys Ser Phe Gly Ser Pro Gly Ser Leu Gly Lys Leu Leu
   355 360 365
   Cys Glu Val Thr Pro Val Ser Gly Gln Glu Arg Phe Val Trp Ser Ser
   370 375 380
   Leu Asp Thr Pro Ser Gln Arg Ser Phe Ser Gly Pro Trp Leu Glu Ala
   385 390 395 400
   Gln Glu Ala Gln Leu Leu Ser Gln Pro Trp Gln Cys Gln Leu Tyr Gln
   405 410 415
   Gly Glu Arg Leu Leu Gly Ala Ala Val Tyr Phe Thr Glu Leu Ser Ser
   420 425 430
   Pro Gly Ala Gln Arg Ser Gly Arg Ala Pro Gly Ala Leu Pro Ala Gly
   435 440 445
   His Leu Leu Leu Phe Leu Thr Leu Gly Val Leu Ser Leu Leu Leu Leu
   450 455 460
   Val Thr Gly Ala Phe Gly Phe His Leu Trp Arg Arg Gln Trp Arg Pro
   465 470 475 480
   Arg Arg Phe Ser Ala Leu Glu Gln Gly Ile His Pro Pro Gln Ala Gln
   485 490 495
   Ser Lys Ile Glu Glu Leu Glu Gln Glu Pro Glu Pro Glu Pro Glu Pro
   500 505 510
   Glu Pro Glu Pro Glu Pro Glu Pro Glu Pro Glu Gln Leu
   515 520 525
<210> 14
   <211> 8
   <212> PRT
   <213> Artificial Sequence
<220>
   <223> Synthetic: LAG-3 Epitope
<400> 14
   Pro Gly His Pro Leu Ala Pro Gly
   1               5
<210> 15
   <211> 8
   <212> PRT
   <213> Artificial Sequence
<220>
   <223> Synthetic: LAG-3 Epitope
<400> 15
   His Pro Ala Ala Pro Ser Ser Trp
   1               5
<210> 16
   <211> 8
   <212> PRT
   <213> Artificial Sequence
<220>
   <223> Synthetic: LAG-3 Epitope
<400> 16
   Pro Ala Ala Pro Ser Ser Trp Gly
   1               5
<210> 17
   <211> 1344
   <212> DNA
   <213> Artificial Sequence
<220>
   <223> Synthetic: Heavy Chain Nucleotide Sequence, Anti-LAG-3 mAb (BMS-986016)
<400> 17
   caggtgcagc tacagcagtg gggcgcagga ctgttgaagc cttcggagac cctgtccctc 60
   acctgcgctg tctatggtgg gtccttcagt gattactact ggaactggat ccgccagccc 120
   ccagggaagg ggctggagtg gattggggaa atcaatcatc gtggaagcac caactccaac 180
   ccgtccctca agagtcgagt caccctatca ctagacacgt ccaagaacca gttctccctg 240
   aagctgaggt ctgtgaccgc cgcggacacg gctgtgtatt actgtgcgtt tggatatagt 300
   gactacgagt acaactggtt cgacccctgg ggccagggaa ccctggtcac cgtctcctca 360
   gctagcacca agggcccatc cgtcttcccc ctggcgccct gctccaggag cacctccgag 420
   agcacagccg ccctgggctg cctggtcaag gactacttcc ccgaaccggt gacggtgtcg 480
   tggaactcag gcgccctgac cagcggcgtg cacaccttcc cggctgtcct acagtcctca 540
   ggactctact ccctcagcag cgtggtgacc gtgccctcca gcagcttggg cacgaagacc 600
   tacacctgca acgtagatca caagcccagc aacaccaagg tggacaagag agttgagtcc 660
   aaatatggtc ccccatgccc accatgccca gcacctgagt tcctgggggg accatcagtc 720
   ttcctgttcc ccccaaaacc caaggacact ctcatgatct cccggacccc tgaggtcacg 780
   tgcgtggtgg tggacgtgag ccaggaagac cccgaggtcc agttcaactg gtacgtggat 840
   ggcgtggagg tgcataatgc caagacaaag ccgcgggagg agcagttcaa cagcacgtac 900
   cgtgtggtca gcgtcctcac cgtcctgcac caggactggc tgaacggcaa ggagtacaag 960
   tgcaaggtct ccaacaaagg cctcccgtcc tccatcgaga aaaccatctc caaagccaaa 1020
   gggcagcccc gagagccaca ggtgtacacc ctgcccccat cccaggagga gatgaccaag 1080
   aaccaggtca gcctgacctg cctggtcaaa ggcttctacc ccagcgacat cgccgtggag 1140
   tgggagagca atgggcagcc ggagaacaac tacaagacca cgcctcccgt gctggactcc 1200
   gacggctcct tcttcctcta cagcaggcta accgtggaca agagcaggtg gcaggagggg 1260
   aatgtcttct catgctccgt gatgcatgag gctctgcaca accactacac acagaagagc 1320
   ctctccctgt ctctgggtaa atga 1344
<210> 18
   <211> 645
   <212> DNA
   <213> Artificial Sequence
<220>
   <223> Synthetic: Light Chain Nucleotide Sequence, Anti-LAG-3 mAb (BMS-986016)
<400> 18
   gaaattgtgt tgacacagtc tccagccacc ctgtctttgt ctccagggga aagagccacc      60
   ctctcctgca gggccagtca gagtattagc agctacttag cctggtacca acagaaacct     120
   ggccaggctc ccaggctcct catctatgat gcatccaaca gggccactgg catcccagcc     180
   aggttcagtg gcagtgggtc tgggacagac ttcactctca ccatcagcag cctagagcct     240
   gaagattttg cagtttatta ctgtcagcag cgtagcaact ggcctctcac ttttggccag     300
   gggaccaacc tggagatcaa acgtacggtg gctgcaccat ctgtcttcat cttcccgcca     360
   tctgatgagc agttgaaatc tggaactgcc tctgttgtgt gcctgctgaa taacttctat     420
   cccagagagg ccaaagtaca gtggaaggtg gataacgccc tccaatcggg taactcccag     480
   gagagtgtca cagagcagga cagcaaggac agcacctaca gcctcagcag caccctgacg     540
   ctgagcaaag cagactacga gaaacacaaa gtctacgcct gcgaagtcac ccatcagggc     600
   ctgagctcgc ccgtcacaaa gagcttcaac aggggagagt gttag                     645